# EUROPÄISCHE PATENTANMELDUNG

(11) **EP 0 926 236 A1**
(43) Veröffentlichungstag der Anmeldung: **30.06.1999**
(21) Anmeldenummer: 98123708.4
(22) Anmeldetag: 12.12.1998
(51) Int. Cl.: C12N 15/12, C07K 14/47, C07K 16/18, C12N 15/11, C12Q 1/68, G01N 33/68, A61K 31/70, A61K 38/17, A61K 39/395

(54) **Bindungspartner für Inhibitoren von cyclinabhängigen Kinasen und ihre Verwendung zur Suche nach Inhibitoren, zur Diagnose oder zur Therapie**

(30) Priorität: 20.12.1997 DE 19756975
(71) Anmelder: Hoechst Marion Roussel Deutschland GmbH, 65929 Frankfurt am Main (DE)
(72) Erfinder: Eilers, Martin, Prof. Dr., 35043 Marburg (DE); Buergin, Andrea, 35037 Marburg (DE); Sedlacek, Hans-Harald, Prof. Dr., 35041 Marburg (DE)

(57) **Zusammenfassung**

Die vorliegende Anmeldung betrifft ein Protein, welches das zelluläre Protein p27 inhibiert und hierdurch die durch p27 bewirkte Hemmung der Proliferation der Zelle aufhebt, Mutanten davon mit z.T. dominant interferierendem Charakter, die entsprechenden dafür kodierenden Nukleinsäuren und die Verwendung von Proteinen und Nukleinsäuren für die Prophylaxe und Therapie von Erkrankungen. Desweiteren werden Nukleinsäurekonstrukte für die Gentherapie von Erkrankungen offenbart, enthaltend o.g. Nukleinsäuren.

SEQUENZPROTOKOLL

(1) ALLGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Germany
(F) POSTLEITZAHL: 65926
(G) TELEPHON: 069-305-3005
(H) TELEFAX: 069-35-7175
(I) TELEX: -

(ii) ANMELDETITEL: Bindungspartner fuer Inhibitoren von
cyclinabhaengigen Kinasen und ihre Verwendung zur Suche
nach Inhibitoren, zur Diagnose oder zur Therapie einer
Erkrankung

(iii) ANZAHL DER SEQUENZEN: 49

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 1958 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..1958

(xi) SEQUENZBESCHREIBUNG: SEQ ID NO: 1:

TCGAATTGGG TACCGGGCCC CCCCTCGAGG TCGACGGTAT CGATAAGCTT GATATCGAAT 60

TCGCGGCCGC TCGAGTTACA AGATGGCGGC CCCGGGCGCT CTCTTCACCG TTCTGTAGCA 120

GCTTCGGGCT GAGCGGATGT CTCTTCTTGT CCTCAGTGTC GGACTCAGAG ACACACGGCT 180

CCCGAGTTCT GCTGATCACG AAGTTCCCGG AGGCGCTCGA CGCACCGGAA TCTCCCAGCG 240

GCCGCGACCG CCGCCTCGGC CCTGCTCGCC GCGGCGCCGG GACTCCAGCG TGATCGGCGG 300

CGGCAGTCAA GGTTCACAAA AATGGCGAAG AGAGTTGCGG AGAAGGAGTT GACTGACAGG 360

AACTGGGATG AGGAAGACGA AGTTGAAGAG ATGGGAACAT TCTCAGTGGC CAGTGAGGAA 420

GTCATGAAGA ACAGAGCCGT AAAGAAGGCA AAGCGCAGAA ACGTTGGATT TGAATCTGAT 480

AGCGGAGGAG CCTTTAAAGG TTTCAAAGGT TTGGTTGTGC CTTCTGGAGG AGGAGGGTTT 540

TCTGGATTTG GTGGCTCTGG AGGAAAGCCT CTGGAAGGAC TGACAAATGG AAACAGCACA 600

GACAATGCCA CGCCCTTCTC CAATGTAAAG ACAGCAGCAG AGCCCAAGGC AGCCTTTGGT 660

TCTTTTGCTG TGAATGGCCC TACTACCTTG GTGGATAAAG TTTCAAATCC AAAAACTAAT 720

GGGGACAGCA ATCAGCCGCC CTCCTCCGGC CCTGCTTCCA GTACCGCCTG CCCTGGGAAT 780

GCCTATCACA AGCAGCTGGC TGGCTTGAAC TGCTCCGTCC GCGATTGGAT AGTGAAGCAC 840

GTGAACACAA ACCCGCTTTG TGACCTGACT CCCATTTTTA AAGACTATGA GAGATACTTG 900

GCGACGATCG AGAAGCAGCT TGAGAATGGA GGCGGCAGCA GTTCTGAGAG CCAGACAGAC 960

AGGGCGACGG CTGGAATGGA GCCTCCTTCC CTTTTTGGTT CAACAAAACT ACAGCAAGAG 1020

TCACCATTTT CATTTCATGG CAACAAAGCG GAGGACACAT CTGAAAAGGT GGAGTTTACA 1080

GCAGAAAAGA AATCGGACGC AGCACAAGGA GCAACAAGTG CCTCGTTTAG TTTCGGCAAG 1140

AAAATTGAGA GCTCGGCTTT GGGCTCGTTA AGCTCTGGCT CCCTAACTGG GTTTTCATTC 1200

TCTGCTGGAA GCTCCAGCTT GTTTGGTAAA GATGCTGCCC AGAGTAAAGC AGCCTCTTCG 1260

CTGTTCTCTG CTAAAGCATC CGAGAGTCCG GCAGGAGGCG GCAGCAGCGA GTGCAGAGAT 1320

GGTGAAGAAG AGGAGAATGA CGAGCCACCC AAGGTAGTGG TGACCGAAGT AAAGGAAGAG 1380

GATGCTTTCT ACTCCAAAAA ATGTAAACTA TTTTACAAGA AAGACAACGA ATTTAAAGAG 1440

AAGGGTGTGG GGACCCTGCA TTTAAAACCC ACAGCAACTC AGAAGACCCA GCTCTTGGTG 1500

CGGGCAGACA CCAACCTAGG CAACATACTG CTGAATGTTC TGATCGCCCC CAACATGCCG 1560

TGCACCCGGA CAGGAAAGAA CAACGTCCTT ATCGTCTGTG TCCCCAACCC CCCACTCGAT 1620

GAGAAGCAGC CCACTCTCCC GGCCACCATG CTGATCCGGG TGAAGACGAG CGAGGATGCC 1680

GATGAATTGC ACAAGATTTT ACTGGAGAAA AAGGATGCCT GAGCACTGAG GCTGACCAAG 1740

GCATGTTGCC ACGTTGCTGC TTCCCCTCCG TCCCTAACTT AGTCACATTC TTTCCTCTTC 1800

TACTGTGACA TTCTGAGAAC TTCTAGGTAA CTTGAACTTT TGTGAGGAAG ATTAAGGCCA 1860

ATAAATCCTT TCAGTGGCGG CCGCGAATTC CTGCAGCCCG GGGGATCCAC TAGTTCTAGA 1920

GCGGCCGCCA CCGCGGTGGA GCTCCAGCTT TTGGGAGA 1958

(2) INFORMATION ZU SEQ ID NO: 2:

(i) SEQUENZ CHARAKTERISTIKA:
(A) LÄNGE: 24 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..24

(xi) SEQUENZBESCHREIBUNG: SEQ ID NO: 2:

Met Ala Lys Arg Val Ala Glu Lys Glu Leu Thr Asp Arg Asn Trp Asp
1               5                   10                  15
Glu Glu Asp Glu Val Glu Glu Met
            20

(2) INFORMATION ZU SEQ ID NO: 3:

(i) SEQUENZ CHARAKTERISTIKA:
(A) LÄNGE: 24 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..24

(xi) SEQUENZBESCHREIBUNG: SEQ ID NO: 3:

Met Ala Lys Arg Val Ala Asp Ala Gln Ile Gln Arg Glu Thr Tyr Asp
1               5                   10                  15

Ser Asn Glu Ser Asp Asp Asp Val
            20

(2) INFORMATION ZU SEQ ID NO: 4:

(i) SEQUENZ CHARAKTERISTIKA:
(A) LÄNGE: 60 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..60

(xi) SEQUENZBESCHREIBUNG: SEQ ID NO: 4:

Asn Gln Pro Pro Ser Ser Gly Pro Ala Ser Ser Thr Ala Cys Pro Gly
1               5                   10                  15

Asn Ala Tyr His Lys Gln Leu Ala Gly Leu Asn Cys Ser Val Arg Asp
            20                  25                  30

Trp Ile Val Lys His Val Asn Thr Asn Pro Leu Cys Asp Leu Thr Pro
        35                  40                  45

Ile Phe Lys Asp Tyr Glu Arg Tyr Leu Ala Thr Ile
    50                  55                  60

(2) INFORMATION ZU SEQ ID NO: 5:

(i) SEQUENZ CHARAKTERISTIKA:
(A) LÄNGE: 60 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..60

(xi) SEQUENZBESCHREIBUNG: SEQ ID NO: 5:

Asn Arg Ala Asp Gly Thr Gly Glu Ala Gln Val Asp Asn Ser Pro Thr
1               5                   10                  15

Thr Glu Ser Asn Ser Arg Leu Lys Ala Leu Asn Leu Gln Phe Lys Ala
            20                  25                  30

Lys Val Asp Asp Leu Val Leu Gly Lys Pro Leu Ala Asp Leu Arg Pro
        35                  40                  45

Leu Phe Thr Arg Tyr Glu Leu Tyr Ile Lys Asn Ile
    50                  55                  60

(2) INFORMATION ZU SEQ ID NO: 6:

(i) SEQUENZ CHARAKTERISTIKA:
(A) LÄNGE: 51 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..51

(xi) SEQUENZBESCHREIBUNG: SEQ ID NO: 6:

Ala Thr Ala Gly Met Glu Pro Pro Ser Leu Phe Gly Ser Thr Lys Leu
1               5                   10                  15

Gln Gln Glu Ser Pro Phe Ser Phe His Gly Asn Lys Ala Glu Asp Thr
            20                  25                  30

Ser Glu Lys Val Glu Phe Thr Ala Glu Lys Lys Ser Asp Ala Ala Gln
        35                  40                  45

Gly Ala Thr
    50

(2) INFORMATION ZU SEQ ID NO: 7:

(i) SEQUENZ CHARAKTERISTIKA:
(A) LÄNGE: 51 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..51

(xi) SEQUENZBESCHREIBUNG: SEQ ID NO: 7:

Ala Asn Ser Ser Thr Ser Pro Ala Pro Ser Ile Pro Ser Thr Gly Phe
1               5                   10                  15

Lys Phe Ser Leu Pro Phe Glu Gln Lys Gly Ser Gln Thr Thr Thr Asn
            20                  25                  30

Asp Ser Lys Glu Glu Ser Thr Thr Glu Ala Thr Gly Asn Glu Ser Gln
        35                  40                  45

Asp Ala Thr
    50

(2) INFORMATION ZU SEQ ID NO: 8:

(i) SEQUENZ CHARAKTERISTIKA:
(A) LÄNGE: 34 Aminosäuren

(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..34

(xi) SEQUENZBESCHREIBUNG: SEQ ID NO: 8:

His Gly Asn Lys Ala Glu Asp Thr Ser Glu Lys Val Glu Phe Thr Ala
1               5                   10                  15

Glu Lys Lys Ser Asp Ala Ala Gln Gly Ala Thr Ser Ala Ser Phe Ser
            20                  25                  30

Phe Gly

(2) INFORMATION ZU SEQ ID NO: 9:

(i) SEQUENZ CHARAKTERISTIKA:
(A) LÄNGE: 34 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..34

(xi) SEQUENZBESCHREIBUNG: SEQ ID NO: 9:

Asn Gly Ser Glu Ser Lys Asp Ser Asp Lys Pro Ser Leu Pro Ser Ala
1               5                   10                  15

Val Asp Gly Glu Asn Asp Lys Lys Glu Ala Thr Lys Pro Ala Phe Ser
            20                  25                  30

Phe Gly

(2) INFORMATION ZU SEQ ID NO: 10:

(i) SEQUENZ CHARAKTERISTIKA:
(A) LÄNGE: 40 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..40

(xi) SEQUENZBESCHREIBUNG: SEQ ID NO: 10:

Leu Gly Asn Ile Leu Leu Asn Val Leu Ile Ala Pro Asn Met Pro Cys
1               5                   10                  15

Thr Arg Thr Gly Lys Asn Asn Val Leu Ile Val Cys Val Pro Asn Pro
            20                  25                  30

Pro Leu Asp Glu Lys Gln Pro Thr
        35                  40

(2) INFORMATION ZU SEQ ID NO: 11:

(i) SEQUENZ CHARAKTERISTIKA:
(A) LÄNGE: 40 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..40

(xi) SEQUENZBESCHREIBUNG: SEQ ID NO: 11:

Met Gly Asn Val Leu Leu Asn Ala Thr Val Val Asp Ser Phe Lys Tyr
1               5                   10                  15

Glu Pro Leu Ala Pro Gly Asn Asp Asn Leu Ile Lys Ala Pro Thr Val
            20                  25                  30

Ala Ala Asp Gly Lys Leu Val Thr
        35                  40

(2) INFORMATION ZU SEQ ID NO: 12:

(i) SEQUENZ CHARAKTERISTIKA:
(A) LÄNGE: 157 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein

(B) LAGE: 1..157

(xi) SEQUENZBESCHREIBUNG: SEQ ID NO: 12:

Asp Ser His Ala Asp His Asp Thr Ser Thr Glu Asn Ala Asp Glu Ser
1               5                   10                  15

Thr Thr His Pro Gln Phe Glu Pro Ile Val Ser Val Pro Glu Gln Glu
            20                  25                  30

Ile Lys Thr Leu Glu Glu Asp Glu Glu Glu Leu Phe Lys Met Arg Ala
        35                  40                  45

Lys Leu Phe Arg Phe Ala Ser Glu Asn Asp Leu Pro Glu Trp Lys Glu
    50                  55                  60

Pro Arg His Gly Asp Val Lys Leu Leu Lys His Lys Glu Lys Gly Thr
65                  70                  75                  80

Ile Arg Leu Leu Met Arg Arg Asp Lys Thr Leu Lys Ile Cys Ala Asn
                85                  90                  95

His Tyr Ile Thr Pro Met Met Glu Leu Lys Pro Asn Ala Gly Ser Asp
            100                 105                 110

Arg Ala Trp Val Trp Asn Thr His Thr Asp Phe Ala Asp Glu Cys Pro
        115                 120                 125

Lys Pro Glu Leu Leu Ala Ile Arg Phe Leu Asn Ala Glu Asn Ala Gln
    130                 135                 140

Lys Phe Lys Thr Lys Phe Glu Glu Cys Arg Lys Glu Ile
145                 150                 155

(2) INFORMATION ZU SEQ ID NO: 13:

(i) SEQUENZ CHARAKTERISTIKA:
(A) LÄNGE: 157 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..157

(xi) SEQUENZBESCHREIBUNG: SEQ ID NO: 13:

Asp Ser His Ala Asp His Asp Thr Ser Thr Glu Asn Ala Asp Glu Ser
1               5                   10                  15

Asn His Asp Pro Gln Phe Glu Pro Ile Val Ser Val Pro Glu Gln Glu
            20                  25                  30

Ile Lys Thr Leu Glu Glu Asp Glu Glu Glu Leu Phe Lys Met Arg Ala
          35                  40                  45

Lys Leu Phe Arg Phe Ala Ser Glu Asn Asp Leu Pro Glu Trp Lys Glu
    50                  55                  60

Arg Gly Thr Gly Asp Val Lys Leu Leu Lys His Lys Glu Lys Gly Thr
65                  70                  75                  80

Ile Arg Leu Leu Met Arg Arg Asp Lys Thr Leu Lys Ile Cys Ala Asn
                85                  90                  95

His Tyr Ile Thr Pro Met Met Glu Leu Lys Pro Asn Ala Gly Ser Asp
            100                 105                 110

Arg Ala Trp Val Trp Asn Thr His Thr Asp Phe Ala Asp Glu Cys Pro
        115                 120                 125

Lys Pro Glu Leu Leu Ala Ile Arg Phe Leu Asn Ala Glu Asn Ala Gln
    130                 135                 140

Lys Phe Lys Thr Lys Phe Glu Glu Cys Arg Lys Glu Ile
145                 150                 155

(2) INFORMATION ZU SEQ ID NO: 14:

     (i) SEQUENZ CHARAKTERISTIKA:
          (A) LÄNGE: 158 Aminosäuren
          (B) ART: Aminosäure
          (C) STRANGFORM: Einzel
          (D) TOPOLOGIE: linear

    (ii) ART DES MOLEKÜLS: Protein

    (ix) MERKMALE:
          (A) NAME/SCHLÜSSEL: Protein
          (B) LAGE: 1..158

    (xi) SEQUENZBESCHREIBUNG: SEQ ID NO: 14:

Ser Gln Thr Thr Thr Asn Asp Ser Lys Glu Glu Ser Thr Thr Glu Ala
1               5                   10                  15

Thr Gly Asn Glu Ser Gln Asp Ala Thr Lys Val Asp Ala Thr Pro Glu
            20                  25                  30

Glu Ser Lys Pro Ile Asn Leu Gln Asn Gly Glu Glu Asp Glu Val Ala
        35                  40                  45

Leu Phe Ser Gln Lys Ala Lys Leu Met Thr Phe Asn Ala Glu Thr Lys
    50                  55                  60

Ser Tyr Asp Ser Arg Gly Val Gly Glu Met Lys Leu Leu Lys Lys Lys
65                  70                  75                  80

Asp Asp Ser Pro Lys Val Arg Leu Leu Cys Arg Ser Asp Gly Met Gly
                85                  90                  95

Asn Val Leu Leu Asn Ala Thr Val Val Asp Ser Phe Lys Tyr Glu Pro
            100                 105                 110

Leu Ala Pro Gly Asn Asp Asn Leu Ile Lys Ala Pro Thr Val Ala Ala
        115                 120                 125

Asp Gly Lys Leu Val Thr Tyr Ile Val Phe Lys Gln Lys Leu Glu Gly
    130                 135                 140

Arg Ser Phe Thr Lys Ala Ile Glu Asp Ala Lys Lys Glu Met
145                 150                 155

(2) INFORMATION ZU SEQ ID NO: 15:

(i) SEQUENZ CHARAKTERISTIKA:
(A) LÄNGE: 160 Aminosäuren
(B) ART: Aminosäure
(C) STRANGFORM: Einzel
(D) TOPOLOGIE: linear

(ii) ART DES MOLEKÜLS: Protein

(ix) Merkmale:
(A) NAME/SCHLÜSSEL: Protein
(B) LAGE: 1..160

(xi) SEQUENZBESCHREIBUNG: SEQ ID NO: 15:

Gln Ser Lys Ala Ala Ser Ser Leu Phe Ser Ala Lys Ala Ser Glu Ser
1               5                   10                  15

Pro Ala Gly Gly Gly Ser Ser Glu Cys Arg Asp Gly Glu Glu Glu Glu
            20                  25                  30

Asn Asp Glu Pro Pro Lys Val Val Val Thr Glu Val Lys Glu Glu Asp
        35                  40                  45

Ala Phe Tyr Ser Lys Lys Cys Lys Leu Phe Tyr Lys Lys Asp Asn Glu
    50                  55                  60

Phe Lys Glu Lys Gly Val Gly Thr Leu His Leu Lys Pro Thr Ala Thr
65                  70                  75                  80

Gln Lys Thr Gln Leu Leu Val Arg Ala Asp Thr Asn Leu Gly Asn Ile
                85                  90                  95

Leu Leu Asn Val Leu Ile Ala Pro Asn Met Pro Cys Thr Arg Thr Gly
            100                 105                 110

Lys Asn Asn Val Leu Ile Val Cys Val Pro Asn Pro Leu Asp Glu Lys
        115                 120                 125

Gln Pro Thr Leu Pro Ala Thr Met Leu Ile Arg Val Lys Thr Ser Glu
    130                 135                 140

Asp Ala Asp Glu Leu His Lys Ile Leu Leu Glu Lys Lys Asp Ala Ala
145                 150                 155                 160

(2) INFORMATION ZU SEQ ID NO: 16:

(i) SEQUENZ CHARAKTERISTIKA:
(A) LÄNGE: 466 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..466

(xi) SEQUENZBESCHREIBUNG: SEQ ID NO: 16:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60

Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Thr Thr Leu Val Asp Lys Val Ser
        115                 120                 125

Asn Pro Lys Thr Asn Gly Asp Ser Asn Gln Pro Pro Ser Ser Gly Pro
    130                 135                 140

Ala Ser Ser Thr Ala Cys Pro Gly Asn Ala Tyr His Lys Gln Leu Ala
145                 150                 155                 160

Gly Leu Asn Cys Ser Val Arg Asp Trp Ile Val Lys His Val Asn Thr
                165                 170                 175

Asn Pro Leu Cys Asp Leu Thr Pro Ile Phe Lys Asp Tyr Glu Arg Tyr
            180                 185                 190

Leu Ala Thr Ile Glu Lys Gln Leu Glu Asn Gly Gly Gly Ser Ser Ser
        195                 200                 205

Glu Ser Gln Thr Asp Arg Ala Thr Ala Gly Met Glu Pro Pro Ser Leu
    210                 215                 220

Phe Gly Ser Thr Lys Leu Gln Gln Glu Ser Pro Phe Ser Phe His Gly
225                 230                 235                 240

Asn Lys Ala Glu Asp Thr Ser Glu Lys Val Glu Phe Thr Ala Glu Lys
                245                 250                 255

Lys Ser Asp Ala Ala Gln Gly Ala Thr Ser Ala Ser Phe Ser Phe Gly
            260                 265                 270

Lys Lys Ile Glu Ser Ser Ala Leu Gly Ser Leu Ser Ser Gly Ser Leu
        275                 280                 285

Thr Gly Phe Ser Phe Ser Ala Gly Ser Ser Ser Leu Phe Gly Lys Asp
    290                 295                 300

Ala Ala Gln Ser Lys Ala Ala Ser Ser Leu Phe Ser Ala Lys Ala Ser
305                 310                 315                 320

Glu Ser Pro Ala Gly Gly Gly Ser Ser Glu Cys Arg Asp Gly Glu Glu
                325                 330                 335

Glu Glu Asn Asp Glu Pro Pro Lys Val Val Val Thr Glu Val Lys Glu
            340                 345                 350

Glu Asp Ala Phe Tyr Ser Lys Lys Cys Lys Leu Phe Tyr Lys Lys Asp
        355                 360                 365

Asn Glu Phe Lys Glu Lys Gly Val Gly Thr Leu His Leu Lys Pro Thr
    370                 375                 380

Ala Thr Gln Lys Thr Gln Leu Leu Val Arg Ala Asp Thr Asn Leu Gly
385                 390                 395                 400

Asn Ile Leu Leu Asn Val Leu Ile Ala Pro Asn Met Pro Cys Thr Arg
                405                 410                 415

Thr Gly Lys Asn Asn Val Leu Ile Val Cys Val Pro Asn Pro Pro Leu
            420                 425                 430

Asp Glu Lys Gln Pro Thr Leu Pro Ala Thr Met Leu Ile Arg Val Lys
        435                 440                 445

Thr Ser Glu Asp Ala Asp Glu Leu His Lys Ile Leu Leu Glu Lys Lys
    450                 455                 460

Asp Ala
465

(2) INFORMATION ZU SEQ ID NO: 17:

    (i) SEQUENZ CHARAKTERISTIKA:
        (A) LÄNGE: 715 Aminosäuren

(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..715

(xi) SEQUENZBESCHREIBUNG: SEQ ID NO: 17:

Met Ala Lys Arg Val Ala Asp Ala Gln Ile Gln Arg Glu Thr Tyr Asp
1               5                   10                  15

Ser Asn Glu Ser Asp Asp Asp Val Thr Pro Ser Thr Lys Val Ala Ser
            20                  25                  30

Ser Ala Val Met Asn Arg Arg Lys Ile Ala Met Pro Lys Arg Arg Met
        35                  40                  45

Ala Phe Lys Pro Phe Gly Ser Ala Lys Ser Asp Glu Thr Lys Gln Ala
    50                  55                  60

Ser Ser Phe Ser Phe Leu Asn Arg Ala Asp Gly Thr Gly Glu Ala Gln
65                  70                  75                  80

Val Asp Asn Ser Pro Thr Thr Glu Ser Asn Ser Arg Leu Lys Ala Leu
                85                  90                  95

Asn Leu Gln Phe Lys Ala Lys Val Asp Asp Leu Val Leu Gly Lys Pro
            100                 105                 110

Leu Ala Asp Leu Arg Pro Leu Phe Thr Arg Tyr Glu Leu Tyr Ile Lys
        115                 120                 125

Asn Ile Leu Glu Ala Pro Val Lys Phe Ile Glu Asn Pro Thr Gln Thr
    130                 135                 140

Lys Gly Asn Asp Ala Lys Pro Ala Lys Val Glu Asp Val Gln Lys Ser
145                 150                 155                 160

Ser Asp Ser Ser Ser Glu Asp Glu Val Lys Val Glu Gly Pro Lys Phe
                165                 170                 175

Thr Ile Asp Ala Lys Pro Pro Ile Ser Asp Ser Val Phe Ser Phe Gly
            180                 185                 190

Pro Lys Lys Glu Asn Arg Lys Lys Asp Glu Ser Asp Ser Glu Asn Asp
        195                 200                 205

Ile Glu Ile Lys Gly Pro Glu Phe Lys Phe Ser Gly Thr Val Ser Ser
    210                 215                 220

Asp Val Phe Lys Leu Asn Pro Ser Thr Asp Lys Asn Glu Lys Lys Thr
225                 230                 235                 240

Glu Thr Asn Ala Lys Pro Phe Ser Phe Ser Ser Ala Thr Ser Thr Thr
                245                 250                 255

Glu Gln Thr Lys Ser Lys Asn Pro Leu Ser Leu Thr Glu Ala Thr Lys
            260                 265                 270

Thr Asn Val Asp Asn Asn Ser Lys Ala Glu Ala Ser Phe Thr Phe Gly
        275                 280                 285

Thr Lys His Ala Ala Asp Ser Gln Asn Asn Lys Pro Ser Phe Val Phe
    290                 295                 300

Gly Gln Ala Ala Ala Lys Pro Ser Leu Glu Lys Ser Ser Phe Thr Phe
305                 310                 315                 320

Gly Ser Thr Thr Ile Glu Lys Lys Asn Asp Glu Asn Ser Thr Ser Asn
                325                 330                 335

Ser Lys Pro Glu Lys Ser Ser Asp Ser Asn Asp Ser Asn Pro Ser Phe
            340                 345                 350

Ser Phe Ser Ile Pro Ser Lys Asn Thr Pro Asp Ala Ser Lys Pro Ser
        355                 360                 365

Phe Asn Phe Gly Val Pro Asn Ser Ser Lys Asn Glu Thr Ser Lys Pro
    370                 375                 380

Val Phe Ser Phe Gly Ala Ala Thr Pro Ser Ala Lys Glu Ala Ser Gln
385                 390                 395                 400

Glu Asp Asp Asn Asn Asn Val Glu Lys Pro Ser Ser Lys Pro Ala Phe
                405                 410                 415

Asn Phe Ile Ser Asn Ala Gly Thr Glu Lys Glu Lys Glu Ser Lys Lys
            420                 425                 430

Asp Ser Lys Pro Ala Phe Ser Phe Gly Ile Ser Asn Gly Ser Glu Ser
        435                 440                 445

Lys Asp Ser Asp Lys Pro Ser Leu Pro Ser Ala Val Asp Gly Glu Asn
    450                 455                 460

Asp Lys Lys Glu Ala Thr Lys Pro Ala Phe Phe Gly Ile Asn Thr Asn
465                 470                 475                 480

Thr Thr Lys Thr Ala Asp Thr Lys Ala Pro Thr Phe Thr Phe Gly Ser
                485                 490                 495

Ser Ala Leu Ala Asp Asn Lys Glu Asp Val Lys Lys Pro Phe Ser Phe
            500                 505                 510

Gly Thr Ser Gln Pro Asn Asn Thr Pro Ser Phe Ser Phe Gly Lys Thr
        515                 520                 525

Thr Ala Asn Leu Pro Ala Asn Ser Ser Thr Ser Pro Ala Pro Ser Ile
    530                 535                 540

Pro Ser Thr Gly Phe Lys Phe Ser Leu Pro Phe Glu Gln Lys Gly Ser
545                 550                 555                 560

Gln Thr Thr Thr Asn Asp Ser Lys Glu Glu Ser Thr Thr Glu Ala Thr
                565                 570                 575

Gly Asn Glu Ser Gln Asp Ala Thr Lys Val Asp Ala Thr Pro Glu Glu
            580                 585                 590

Ser Lys Pro Ile Asn Leu Gln Asn Gly Glu Glu Asp Glu Val Ala Leu
        595                 600                 605

Phe Ser Lys Ala Lys Leu Met Thr Phe Asn Ala Glu Thr Lys Ser Tyr
    610                 615                 620

Asp Ser Arg Gly Val Gly Glu Met Lys Leu Leu Lys Lys Lys Asp Asp
625                 630                 635                 640

Pro Ser Lys Val Arg Leu Leu Cys Arg Ser Asp Gly Met Gly Asn Val
                645                 650                 655

Leu Leu Asn Ala Thr Val Val Asp Ser Phe Lys Tyr Glu Pro Leu Ala
            660                 665                 670

Pro Gly Asn Asp Asn Leu Ile Lys Ala Pro Thr Val Ala Ala Asp Gly
        675                 680                 685

Lys Thr Tyr Ile Val Lys Phe Lys Gln Lys Glu Glu Gly Arg Ser Phe
    690                 695                 700

Thr Lys Ala Ile Glu Asp Ala Lys Lys Glu Lys
705                 710                 715

(2) INFORMATION ZU SEQ ID NO: 18:
(i) SEQUENZ CHARAKTERISTIKA:
(A) LÄNGE: 335 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..335

(xi) SEQUENZBESCHREIBUNG: SEQ ID NO: 18:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60
Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Phe Thr Ala Glu Lys Lys Ser Asp
        115                 120                 125

Ala Ala Gln Gly Ala Thr Ser Ala Ser Phe Ser Phe Gly Lys Lys Ile
    130                 135                 140

Glu Ser Ser Ala Leu Gly Ser Leu Ser Ser Gly Ser Leu Thr Gly Phe
145                 150                 155                 160

Ser Phe Ser Ala Gly Ser Ser Ser Leu Phe Gly Lys Asp Ala Ala Gln
                165                 170                 175

Ser Lys Ala Ala Ser Ser Leu Phe Ser Ala Lys Ala Ser Glu Ser Pro
            180                 185                 190

Ala Gly Gly Gly Ser Ser Glu Cys Arg Asp Gly Glu Glu Glu Glu Asn
        195                 200                 205

Asp Glu Pro Pro Lys Val Val Val Thr Glu Val Lys Glu Glu Asp Ala
    210                 215                 220

Phe Tyr Ser Lys Lys Cys Lys Leu Phe Tyr Lys Lys Asp Asn Glu Phe
225                 230                 235                 240

Lys Glu Lys Gly Val Gly Thr Leu His Leu Lys Pro Thr Ala Thr Gln
                245                 250                 255

Lys Thr Gln Leu Leu Val Arg Ala Asp Thr Asn Leu Gly Asn Ile Leu
            260                 265                 270

Leu Asn Val Leu Ile Ala Pro Asn Met Pro Cys Thr Arg Thr Gly Lys
        275                 280                 285

Asn Asn Val Leu Ile Val Cys Val Pro Asn Pro Pro Leu Asp Glu Lys
    290                 295                 300

Gln Pro Thr Leu Pro Ala Thr Met Leu Ile Arg Val Lys Thr Ser Glu
305                 310                 315                 320

Asp Ala Asp Glu Leu His Lys Ile Leu Leu Glu Lys Lys Asp Ala
                325                 330                 335

(2) INFORMATION ZU SEQ ID NO: 19:

    (i) SEQUENZ CHARAKTERISTIKA:
        (A) LÄNGE: 310 Aminosäuren
        (B) ART: Aminosäure
        (C) STRANGFORM: Einzel
        (D) TOPOLOGIE: linear

    (ii) ART DES MOLEKÜLS: Protein

(ix) MERKMALE:
(A) NAME/SCHLÜSSEL: Protein
(B) LAGE: 1..310

(xi) SEQUENZBESCHREIBUNG: SEQ ID NO: 19:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60

Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Thr Thr Leu Val Asp Lys Val Ser
        115                 120                 125

Asn Pro Lys Thr Asn Gly Asp Ser Asn Gln Pro Pro Ser Ser Gly Pro
    130                 135                 140

Ala Ser Ser Thr Ala Cys Pro Gly Asn Ala Tyr His Lys Gln Leu Ala
145                 150                 155                 160

Gly Leu Asn Cys Ser Val Arg Asp Trp Ile Val Lys His Val Asn Ile
                165                 170                 175

Asn Pro Leu Cys Asp Leu Thr Pro Ile Phe Lys Asp Tyr Glu Arg Tyr
            180                 185                 190

Leu Ala Thr Ile Glu Lys Gln Leu Glu Asn Gly Gly Gly Ser Ser Ser
        195                 200                 205

Glu Ser Gln Thr Asp Arg Ala Thr Ala Gly Met Glu Pro Pro Ser Leu
    210                 215                 220

Phe Gly Ser Thr Lys Leu Gln Gln Glu Ser Pro Phe Ser Phe His Gly
225                 230                 235                 240

Asn Lys Ala Glu Asp Thr Ser Glu Lys Val Glu Phe Thr Ala Glu Lys
                245                 250                 255

Lys Ser Asp Ala Ala Gln Gly Ala Thr Ser Ala Ser Phe Ser Phe Gly
            260                 265                 270

Lys Lys Ile Glu Ser Ser Ala Leu Gly Ser Leu Ser Ser Gly Ser Leu
        275                 280                 285

Thr Gly Phe Ser Phe Ser Ala Gly Ser Ser Ser Leu Phe Gly Lys Asp
    290                 295                 300

Ala Ala Glu Lys Glu Leu
305                 310

(2) INFORMATION ZU SEQ ID NO: 20:

(i) SEQUENZ CHARAKTERISTIKA:
(A) LÄNGE: 132 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..132

(xi) SEQUENZBESCHREIBUNG: SEQ ID NO: 20:

Thr Thr Leu Val Asp Lys Val Ser Asn Pro Lys Thr Asn Gly Asp Ser
1               5                   10                  15

Asn Gln Pro Pro Ser Ser Gly Pro Ala Ser Ser Thr Ala Cys Pro Gly
            20                  25                  30

Asn Ala Tyr His Lys Gln Leu Ala Gly Leu Asn Cys Ser Val Arg Asp
        35                  40                  45

Trp Ile Val Lys His Val Asn Thr Asn Pro Leu Cys Asp Leu Thr Pro
    50                  55                  60

Ile Phe Lys Asp Tyr Glu Arg Tyr Leu Ala Thr Ile Glu Lys Gln Leu
65                  70                  75                  80

Glu Asn Gly Gly Gly Ser Ser Ser Glu Ser Gln Thr Asp Arg Ala Thr
                85                  90                  95

Ala Gly Met Glu Pro Pro Ser Leu Phe Gly Ser Thr Lys Leu Gln Gln
            100                 105                 110

Glu Ser Pro Phe Ser Phe His Gly Asn Lys Ala Glu Asp Thr Ser Glu
        115                 120                 125

Lys Val Glu Phe
    130

(2) INFORMATION ZU SEQ ID NO: 21:

(i) SEQUENZ CHARAKTERISTIKA:

(A) LÄNGE: 22 Basenpaare
(B) ART: Nukleinsäure
(C) STRANGFORM: Einzel
(D) TOPOLOGIE: linear

(ii) ART DES MOLEKÜLS: DNS

(ix) MERKMALE:
(A) NAME/SCHLÜSSEL: exon
(B) LAGE: 1..22

(xi) SEQUENZBESCHREIBUNG: SEQ ID NO: 21:

AGAAAGCAAA GCGCAGAAAT GT 22

(2) INFORMATION ZU SEQ ID NO: 22:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 22:
CAAATCCAGA AAAGCGTCCT C 21

(2) INFORMATION ZU SEQ ID NO: 23:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..24

(xi) SEQUENZBESCHREIBUNG: SEQ ID NO: 23:

TGAAGAATAG AGCCATAAAG AAAG 24

(2) INFORMATION ZU SEQ ID NO: 24:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 24:

AGAAAAGCGT CCTCCTCCAG A 21

(2) INFORMATION ZU SEQ ID NO: 25:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS

(ix) MERKMALE:
(A) NAME/SCHLÜSSEL: exon
(B) LAGE: 1..24

(xi) SEQUENZBESCHREIBUNG: SEQ ID NO: 25:

TGAAGAATAG AGCCATAAAG AAAG 24

(2) INFORMATION ZU SEQ ID NO: 26:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 26:

AGCGCCACTA ACCAAATCCA GA 22

(2) INFORMATION ZU SEQ ID NO: 27:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 27:

AGAAAGCAAA GCGCAGAAAT GT 22

(2) INFORMATION ZU SEQ ID NO: 28:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 28:

GAAAAGCGTC CTCCTCCAGA AG 22

(2) INFORMATION ZU SEQ ID NO: 29:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 29:

GAAAGCAAAG CGCAGAAATG TT 22

(2) INFORMATION ZU SEQ ID NO: 30:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 30:

CTCCAGCGCC ACTACCAAAT C 21

(2) INFORMATION ZU SEQ ID NO: 31:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 31:

CCCGCACGGA GCAGTTCAAG 20

(2) INFORMATION ZU SEQ ID NO: 32:
(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 32:

GCAGCGGCAG ATCCCAAGGT AG 22

(2) INFORMATION ZU SEQ ID NO: 33:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 33:

GGCATCCTTT TTCTCCA 17

(2) INFORMATION ZU SEQ ID NO: 34:

(i) SEQUENZ CHARAKTERISTIKA:
(A) LÄNGE: 22 Basenpaare
(B) ART: Nukleinsäure
(C) STRANGFORM: Einzel
(D)TOPOLOGIE:linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 34:

CGTTCTTATC GTCTCTGTGT TC 22

(2) INFORMATION ZU SEQ ID NO: 35:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon

(B) LAGE: 1..19

(xi) SEQUENZBESCHREIBUNG: SEQ ID NO: 35:

GCATCCTTTT TCTCCAGTA 19

(2) INFORMATION ZU SEQ ID NO: 36:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 36:

TGTTCCAAAT CCACCAAT 18

(2) INFORMATION ZU SEQ ID NO: 37:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..19

(xi) SEQUENZBESCHREIBUNG: SEQ ID NO: 37:

GTCTGCATCC TCGCTGGTT 19

(2) INFORMATION ZU SEQ ID NO: 38:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 38:

CGTTCTTATC GTCTGTGTTC C 21

(2) INFORMATION ZU SEQ ID NO: 39:
(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 39:

TTTTACCCGA ATCAACAT 18

(2) INFORMATION ZU SEQ ID NO: 40:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 40:

GTACGCGAAC AGGGAAGAAT A 21

(2) ANGABEN ZU SEQ ID NO: 41:

(i) SEQUENZKENNZEICHEN:
(A) LÄNGE: 212 Aminosäuren
(B) ART: Aminosäure
(C) STRANGFORM: Einzelstrang
(D) TOPOLOGIE: linear
(ii) ART DES MOLEKÜLS: Protein

(ix) MERKMAL:
(A) NAME/SCHLÜSSEL: Protein
(B) LAGE:1..212

(xi) SEQUENZBESCHREIBUNG: SEQ ID NO: 41:

Arg Val Ser Asn Gly Ser Pro Ser Leu Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln Arg Lys Trp Asn Phe Asp Phe Gln Asn His Lys
    50                  55                  60

Pro Leu Glu Gly Arg Tyr Glu Trp Gln Glu Val Glu Arg Gly Ser Leu
65                  70                  75                  80

Pro Glu Phe Tyr Tyr Arg Pro Pro Arg Pro Pro Lys Ser Ala Cys Lys
                85                  90                  95

Val Leu Ala Gln Glu Ser Gln Asp Val Ser Gly Ser Arg Gln Ala Val
            100                 105                 110

Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Leu Arg Arg Gln
            180                 185                 190

Thr Arg Val Asp Leu Gln Pro Ser Phe Arg Ala Asn Phe Leu Phe Met
        195                 200                 205
Ile Phe Ile Lys
    210

(2) ANGABEN ZU SEQ ID NO: 42:

(i) SEQUENZKENNZEICHEN:
(A) LÄNGE: 183 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..183

(xi) SEQUENZBESCHREIBUNG: SEQ ID NO: 42:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Arg Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Arg Pro Pro Cys Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Asn Gln Ala Gly
        115                 120                 125

Leu Ala Glu Gln Cys Pro Gly Met Arg Lys Arg Pro Ala Ala Glu Asp
    130                 135                 140

Ser Ser Ser Gln Ile Lys Arg Ala Asn Arg Thr Glu Glu Asn Val Ser
145                 150                 155                 160

Asp Gly Ser Pro Asn Ala Gly Thr Val Glu Gln Thr Pro Lys Lys Pro
                165                 170                 175

Gly Leu Arg Arg Gln Thr Arg
            180

(2) ANGABEN ZU SEQ ID NO: 43:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 43:

Arg Val Ser Asn Gly Ser Pro Ser Pro Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln His Lys Trp Asn Phe Asp Phe Gln Asn His Arg
    50                  55                  60

Pro Leu Glu Gly Arg Tyr Glu Trp Gln Glu Val Glu Arg Gly Ser Leu
65                  70                  75                  80

Pro Glu Phe Tyr Tyr Arg Pro Pro Arg Pro Pro Lys Ser Ala Cys Lys
                85                  90                  95

Val Leu Ala Gln Glu Ser Gln Asp Val Ser Gly Ser Arg Gln Ala Val
            100                 105                 110
Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Pro Arg Arg Gln
            180                 185                 190

Thr Arg Val Asp Leu Gln Pro
        195

(2) ANGABEN ZU SEQ ID NO: 44:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 44:

Arg Val Ser Asn Gly Ser Pro Ser Leu Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln Arg Lys Trp Asn Phe Asp Phe Gln Asn His Lys
    50                  55                  60
Pro Leu Glu Gly Arg Tyr Glu Trp Gln Glu Val Glu Arg Gly Ser Leu
65                  70                  75                  80

Pro Glu Phe Tyr Tyr Gly Pro Pro Arg Pro Pro Lys Ser Ala Cys Lys
                85                  90                  95

Val Leu Ala Gln Glu Ser Gln Asp Val Gly Gly Ser Arg Gln Ala Val
            100                 105                 110

Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Leu Arg Arg Gln
            180                 185                 190

Thr Arg Val Asp Leu Gln Pro
        195

(2) ANGABEN ZU SEQ ID NO: 45:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 45:

Val Ser Asn Gly Ser Pro Ser Leu Glu Arg Met Asp Ala Arg Gln Ala
1               5                   10                  15

Asp His Pro Lys Pro Ser Ala Cys Arg Asn Leu Phe Gly Pro Val Asn
            20                  25                  30

His Gly Glu Leu Thr Arg Asp Leu Glu Lys His Cys Arg Asp Met Glu
        35                  40                  45

Glu Ala Ser Gln Arg Lys Trp Asn Phe Asp Phe Gln Asn His Lys Pro
    50                  55                  60

Leu Glu Gly Arg Tyr Glu Trp Gln Glu Val Glu Arg Gly Ser Leu Pro
65                  70                  75                  80

Glu Phe Tyr Tyr Gly Pro Pro Arg Pro Pro Lys Ser Ala Cys Lys Val
                85                  90                  95

Leu Ala Gln Glu Ser Gln Asp Val Ser Gly Ser Arg Gln Ala Val Pro
            100                 105                 110

Leu Ile Gly Ser Gln Ala Asn Ser Glu Asp Arg His Leu Val Asp Gln
        115                 120                 125

Met Pro Asp Ser Ser Asp Asn Gln Ala Gly Leu Ala Glu Gln Cys Pro
    130                 135                 140

Gly Met Arg Lys Arg Pro Ala Ala Glu Asp Ser Ser Ser Gln Ile Lys
145                 150                 155                 160

Arg Ala Asn Arg Thr Glu Glu Asn Val Ser Asp Gly Ser Pro Asn Ala
                165                 170                 175

Gly Thr Val Glu Gln Thr Pro Lys Lys Pro Gly Leu Arg Arg Gln Thr
            180                 185                 190

Arg Val Asp Leu Gln Pro Ser
        195

(2) ANGABEN ZU SEQ ID NO: 46:

    (i) SEQUENZKENNZEICHEN:
        (A) LÄNGE: 220 Aminosäuren
        (B) ART: Aminosäure
        (C) STRANGFORM: Einzelstrang
        (D) TOPOLOGIE: linear
   (ii) ART DES MOLEKÜLS: Protein

   (ix) MERKMAL:
        (A) NAME/SCHLÜSSEL: Protein
        (B) LAGE:1..220

   (xi) SEQUENZBESCHREIBUNG: SEQ ID NO: 46:

Asn Val Arg Val Ser Asn Gly Ser Pro Ser Leu Glu Arg Met Asp Ala
1               5                   10                  15

Arg Gln Ala Asp His Pro Pro Ser Ala Cys Arg Asn Leu Phe Gly Pro
            20                  25                  30

Val Asn His Gly Glu Leu Thr Arg Asp Leu Glu Lys His Cys Arg Asp
     35                  40                  45

Met Glu Glu Ala Ser Gln Arg Lys Trp Asn Phe Asp Phe Gln Asn His
    50                  55                  60

Lys Pro Leu Glu Gly Arg Tyr Glu Trp Gln Glu Val Glu Arg Gly Ser
65                  70                  75                  80

Leu Pro Glu Phe Tyr Tyr Arg Pro Pro Arg Pro Pro Lys Ser Ala Cys
                85                  90                  95

Lys Val Pro Ala Gln Glu Ser Gln Asp Val Ser Gly Ser Arg Gln Ala
            100                 105                 110

Val Pro Leu Ile Gly Ser Gln Ala Asn Ser Glu Asp Arg His Leu Val
        115                 120                 125

Asp Gln Met Pro Asp Ser Ser Asp Asn Gln Ala Gly Leu Ala Glu Gln
    130                 135                 140

Cys Pro Gly Met Arg Lys Arg Pro Ala Ala Glu Asp Ser Ser Ser Gln
145                 150                 155                 160

Ile Lys Arg Ala Asn Arg Thr Glu Glu Asn Val Ser Asp Gly Ser Pro
                165                 170                 175

Asn Ala Gly Thr Val Glu Gln Thr Pro Lys Lys Pro Gly Leu Arg Arg
            180                 185                 190

Gln Thr Arg Val Asp Leu Gln Pro Ser Phe Arg Ala Asn Phe Leu Phe
        195                 200                 205

Met Ile Phe Ile Ile Lys Val Ile Lys Lys Ile Ser
    210                 215                 220

(2) ANGABEN ZU SEQ ID NO: 47:

(i) SEQUENZKENNZEICHEN:
(A) LÄNGE: 183 Aminosäuren
(B) ART: Aminosäure
(C) STRANGFORM: Einzelstrang
(D) TOPOLOGIE: linear

(ii) ART DES MOLEKÜLS: Protein
(ix) MERKMAL:
(A) NAME/SCHLÜSSEL: Protein
(B) LAGE:1..183

(xi) SEQUENZBESCHREIBUNG: SEQ ID NO: 47:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Gln Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Arg Pro Pro Arg Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Asn Gln Ala Gly
        115                 120                 125

Leu Ala Glu Gln Cys Pro Gly Met Arg Lys Arg Pro Ala Ala Glu Asp
    130                 135                 140

Ser Ser Ser Gln Ile Lys Arg Ala Asn Arg Thr Glu Glu Asn Val Ser
145                 150                 155                 160

Asp Gly Ser Pro Asn Ala Gly Thr Val Glu Gln Thr Pro Lys Lys Pro
                165                 170                 175

Gly Leu Arg Arg Gln Thr Arg
            180

(2) ANGABEN ZU SEQ ID NO: 48:

(i) SEQUENZKENNZEICHEN:
(A) LÄNGE: 138 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..138

(xi) SEQUENZBESCHREIBUNG: SEQ ID NO: 48:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Arg Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Gly Pro Pro Arg Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Ser Gln Ala Gly
        115                 120                 125
Leu Ala Glu Gln Cys Pro Gly Met Arg Lys
    130                 135

(2) ANGABEN ZU SEQ ID NO: 49:

(i) SEQUENZKENNZEICHEN:
(A) LÄNGE: 197 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..197

(xi) SEQUENZBESCHREIBUNG: SEQ ID NO: 49:

Met Ser Asn Val Arg Val Ser Asn Gly Ser Pro Ser Leu Glu Arg Met
1               5                   10                  15

Asp Ala Arg Gln Ala Asp His Pro Lys Pro Ser Ala Cys Arg Asn Leu
            20                  25                  30

Phe Gly Pro Val Asn His Glu Glu Leu Thr Arg Asp Leu Glu Lys His
        35                  40                  45

Cys Arg Asp Met Glu Glu Ala Ser Gln Arg Lys Trp Asn Phe Asp Phe
    50                  55                  60

Gln Asn His Lys Pro Leu Glu Gly Arg Tyr Glu Trp Gln Glu Val Glu
65                  70                  75                  80

Arg Gly Ser Leu Pro Glu Phe Tyr Tyr Arg Pro Pro Arg Pro Pro Lys
                85                  90                  95

Ser Ala Cys Lys Val Leu Ala Gln Glu Ser Gln Asp Val Ser Gly Ser
            100                 105                 110

Arg Gln Ala Val Pro Leu Ile Gly Ser Gln Ala Asn Ser Glu Asp Arg
        115                 120                 125

His Leu Val Asp Gln Met Pro Asp Ser Ser Asp Asn Gln Ala Gly Leu
    130                 135                 140

Ala Glu Gln Cys Pro Gly Met Arg Lys Arg Pro Ala Ala Glu Asp Ser
145                 150                 155                 160

Ser Ser Gln Asn Lys Arg Ala Asn Arg Thr Glu Glu Asn Val Ser Asp
                165                 170                 175

Gly Ser Pro Asn Ala Gly Thr Val Glu Gln Thr Pro Lys Lys Pro Gly
            180                 185                 190

Leu Arg Arg Gln Thr
        195

## Beschreibung

Die vorliegende Anmeldung betrifft ein Protein, welches das zelluläre Protein p27 inhibiert und hierdurch die durch p27 bewirkte Hemmung der Proliferation der Zelle aufhebt, Mutanten davon mit z.T. dominant interferierenden Charakter, die entsprechenden dafür kodierenden Nukleinsäuren und die Verwendung von Protein und Nukleinsäuren für die Prophylaxe und Therapie von Erkrankungen.

### A) Einführung

Der Zellzyklus eukaryotischer Zellen wird gesteuert durch cyclinabhängige Kinasen ("cyclin-dependent kinases" = cdk's); dies sind Kinasen, die für ihre Aktivität eine regulatorische Untereinheit ("Cyclin") benötigen. Verschiedene Prozesse im Zellzyklus (wie zum Beispiel der Replikation, Eintritt in die Mitose) werden durch verschiedene cdk's gesteuert (Morgan, Nature 374, 131 (1995)). Hierbei ist die Aktivität cyclinabhängiger Kinasen hochreguliert. Dabei gibt es interne Steuerungsmechanismen, die zum Beispiel den Eintritt in die Mitose verhindern, so lange die DNA nicht komplett repliziert ist. Die Steuerung durch externe Faktoren, wie zum Beispiel Wachstumsfaktoren, findet ausschließlich vor dem Beginn der DNA-Replikation statt, Replikation wird durch aktive Cyclin E/cdk2 Komplexe initiiert.

Neben der Menge an Cyclin an der Kinase-Untereinheit, wird die Aktivität cyclinabhängiger Kinasen durch kleine Inhibitorproteine reguliert (Sherr und Roberts, Gene Dev 9, 1149 (1995)). Beispielsweise sind für Cyclin E/cdk2 zwei Inhibitoren, die nach ihrer Größe als p21 und als p27 bezeichnet werden, entscheidend. In Zellen, die hohe Mengen an p27 exprimieren, ist die Cyclin E/cdk2 Kinase normalerweise inaktiv und der Eintritt in die DNA-Replikation ist blockiert.

In vielen humanen Tumoren sind positive Zellzyklusregulatoren überexprimiert oder zumindest konstitutiv exprimiert (Sherr, Science 274, 1672 (1996)), negative Regulatoren aber häufig mutiert oder schwach exprimiert (Fero et al., Cell 85, 733 (1996)). Es gibt spezifische Korrelationen: beispielsweise findet man in vielen Nackentumoren die Überexpression des Cyclin D1-Genes. Daher besteht die Hoffnung, daß cyclinabhängige Kinasen und ihre Funktion Zielstrukturen bei der Suche nach neuen, selektiv wirksamen, antiproliferativ wirkenden Substanzen sein könnten.

Das Gen für das p27 Protein ist seit einigen Jahren bekannt (K. Polyak et al. Cell 78, 59-66 (1994)) und in der Genbank zugänglich [murines p27; Zugangsnummer (Accession Number) K 09968; humanes p27: K 10906]. Trotz intensiver Suche wurden bisher Mutationen im p27 Gen in menschlichen Tumoren nicht gefunden. Dies ist um so überraschender, weil Mäuse, in denen das Gen für p27 inaktiviert worden ist, einen Phänotyp mit multiplen Dysplasien und erhöhter Tumorinzidenz zeigen (Fero et al., Cell 85, 733 (1996), Kiyokawa et al., Cell 85, 721 (1996)). Statt dessen wird offensichtlich die Funktion von p27 im wesentlichen posttranskriptionell reguliert.

So wird p27 durch Proteolyse abgebaut; dies geschieht auch zu Beginn der DNA-Replikation von normalen Zellen. Die Fähigkeit, p27 proteolytisch abzubauen, ist in vielen Tumoren deutlich gegenüber dem Normalgewebe erhöht und dieses scheint direkt mit einer ungünstigen Prognose (Loda et al., Nature Med 3, 231 (1997)) zu korrelieren.

Es muß aber noch weitere Mechanismen geben, die zur Inaktivierung von p27 führen können. Zum Beispiel wird nach der Transformation von Zellen durch das Myc-Onkogen p27 erst funktionell inaktiviert (d.h., es bindet nicht mehr an Cyclin/cdk Komplexe), aber erst viel später abgebaut. Bislang ist unerklärlich, warum bei einer Reihe von zum Beispiel Brusttumoren hohe Mengen von p27 Proteinen exprimiert werden, und diese Tumoren trotzdem sehr schnell wachsen. Die Mechanismen, die p27 in diesen Tumoren inaktivieren, sind bislang unbekannt (Fredersdorf et al., Proc. Natl. Acad. Sci. USA 94, 6380 (1997)).

Es besteht demgemäß ein großes Interesse der Fachwelt, Mechanismen bzw. Stoffe aufzufinden, die für die Inaktivierung von p27 in Tumoren verantwortlich sind. Mit der vorliegenden Erfindung wurde dieses Problem gelöst. Das in der Anmeldung beschriebene Protein p163 kann p27 binden, die Funktion von p27 inhibieren und p27 im Zytoplasma der Proteolyse zuführen.

### B) Allgemeine Beschreibung der Erfindung

### 1) Das Protein p163 und seine Nukleinsäuresequenz

Gegenstand der Erfindung ist ein neues Protein, genannt p163, welches p27 bindet, hierdurch in seiner Funktion inhibiert und in das Zytoplasma der Proteolyse zuführt. Die Aminosäuresequenz dieses Proteins wurde ermittelt.

Das Protein p163 bindet spezifisch an p27 und nicht an andere Proteine wie Myc, Max, Bcl-6 oder Prothymosin-α.

Gegenstand der Erfindung ist desweiteren eine Nukleotidsequenz, welche für das Protein p163 kodiert. Diese Nukleotidsequenz wurde für das Mäuse-p163 Protein und durch Sequenzvergleich auch für das humane p163 Protein ermittelt.

### 2) Dominant negative p163 Analoga

Gegenstand dieser Erfindung sind desweiteren Nukleotidsequenzen, welche für Teilpeptide von p163 und/oder für p163 analoge, dominant negativ wirkende Proteine kodieren, d.h. die die Bindung des natürlichen Proteins p163 an das Protein p27 inhibieren, ohne die Bindung des p27 Proteins an Cyclin E/cdk2 und die hierdurch bedingte Inhibition von Cyclin E/cdk2 wesentlich zu beeinträchtigen.

Gegenstand der Erfindung ist desweiteren die Verwendung von Nukleinsäuresequenzen, die für dominant negative Mutanten des p163 Proteins oder Teilsequenzen hiervon kodieren, wobei die Verwendung beinhaltet die Einführung dieser Nukleinsäuresequenzen in eine Zielzelle und die Inhibition der Bindung zwischen dem zielzelleigenen p163 Protein und dem zielzelleigenen p27 Protein, so daß die Proliferation der Zielzelle durch das freiwerdende zielzelleigene p27 Protein gehemmt wird.

### 3) Peptide, die p163 hemmen

Gegenstand dieser Erfindung sind jedoch auch Nukleotidsequenzen, welche für Proteine oder Peptide kodieren, die die Funktion von p163 hemmen. Hierzu gehören Proteine oder Peptide, die an die Bindestelle des p163 Proteins für das p27 Protein oder an die Bindestelle des p163 Proteins für das Ran (Loeb et al. Mol. Cell. Biol 4, 209-222 (1993)) binden und hierdurch das zellinterne p163 Protein hemmen.

Zu diesen Proteinen gehören Peptide, die der Bindestelle des p27 Proteins oder des Ran Proteins für das p163 Protein entsprechen. Zu diesen Proteinen gehören des weiteren Antikörper oder Antikörperspaltprodukte, wie F(ab)₂ Fab, Fv und s.c. Fv mit Spezifität gegen das Protein p163, insbesondere gegen dessen Bindestelle für das p27 Protein.

Gegenstand der Erfindung ist des weiteren die Verwendung von Nukleinsäuresequenzen kodierend für derartige inhibierende Proteine oder Peptide, wobei die Verwendung beinhaltet die Einführung dieser Nukleinsäuresequenzen in eine Zielzelle und die Inhibition der Bindung zwischen dem zielzelleigenen p163 Protein und dem zielzelleigenen p27 Protein oder dem zielzelleigenen Ran Protein, so daß die Proliferation der Zielzelle durch das freiwerdende zielzelleigene p27 Protein gehemmt wird.

### 4) Verwendung von p163 zur Stimulation der Zellteilung

Gegenstand dieser Erfindung ist des weiteren die Verwendung der Nukleotidsequenz kodierend für das p163 Protein oder das p163 Protein selbst zur Hemmung des zellinternen p27 Proteins und damit zur Stimulation der Zellteilung einer Zielzelle.

### 5) Inhibition der Transkription und Translation von p163

Gegenstand der Erfindung sind jedoch auch Nukleinsäuresequenzen, welche die Transkription und/oder Translation der zielzelleigenen Nukleinsäuresequenz kodierend für das p163 Protein hemmen und hierdurch zu einem Freiwerden des p27 Proteins und damit zu einer Inhibition der Proliferation der Zielzelle führen. Derartige erfindungsgemäße Nukleinsäuresequenzen können beispielsweise antisense (Triple-)DNA, antisense RNA und/oder Ribozyme darstellen.

### 6) Testsysteme zur Findung von p163 Inhibitoren

Gegenstand der Erfindung ist des weiteren die Verwendung der Nukleinsäuresequenz kodierend für das p163 Protein oder des p163 Proteins oder Teilsequenzen hiervon für Testsysteme, in denen nach Inhibitoren der Bindung zwischen dem p163 Protein und dem p27 Protein oder dem Ran Protein gesucht werden und die Verwendung der in diesen Testsystemen gefundenen Inhibitoren des p163 Proteins für die Inhibition der Proliferation einer Zielzelle.

### 7) Nachweis von p163 zur Diagnose einer Erkrankung

Gegenstand der Erfindung ist des weiteren die Verwendung von Nukleinsäurekonstrukten kodierend für das p163 Protein oder für Teilsequenzen des p163 Proteins oder von Nukleinsäuresequenzen oder Proteinsequenzen, welche an diese Nukleinsäuresequenzen für das p163 Protein binden oder von Proteinen, welche an das p163 Protein binden zum Nachweis von Nukleinsäuren kodierend für p163 oder zum Nachweis des p163 Proteins in einer Zelle oder einem Gewebe oder einer Körperflüssigkeit zum Zwecke der Ermittlung des Proliferationszustandes einer Zelle oder eines Gewebes oder der Diagnose eines Krankheitszustandes.

### C) Nähere Beschreibung der Erfindung

### 1) Charakterisierung des p163 Proteins und der das p163 Protein kodierenden Nukleinsäuresequenz

Mit Hilfe der "two-hybrid-Technologie" (Fields und Song, Nature 340, 245 (1989)) wurde das p163 Proteins als ein neues Partnerprotein von p27 entdeckt. Hierzu wurde die gesamte kodierende Sequenz des murinen p27 Proteins mit Hilfe von PCR in den Hefevektor pGBT10 (Clontech) in-frame mit der DNA-bindenden Domäne des Transkriptionsfaktors GAL4 kloniert. Der Hefestamm Hf7c (Fa. Clontech Heidelberg, Matchmaker Two Hybrid, K 1605-1) wurde mit diesem Vektor transformiert, tryptophan-auxotrophe Kolonien isoliert und die Expression der korrekten Fusionsproteine im Western Blot mit Hilfe spezifischer Antikörper gegen GAL4 und p27 nachgewiesen.

Dieser Stamm wurde in einer zweiten Transformation mit einer VP16-getaggten cDNA-Bibliothek aus Mausembryonen transformiert (Vojtek et al., Cell 74, 205 (1993)). 400 Kolonien, die histidin-auxotroph in Gegenwart von 15 nM Aminotriazol waren, wurden weiter analysiert. 70 dieser Klone wurden sequenziert; sie kodierten alle für verschiedene D-Typ Cycline; das sind bekannte Interaktionspartner von p27. Mit Hilfe von Southern Blots wurde gezeigt, daß 390 der 400 resistenten Klone für D-Cycline kodierten. Zwei der verbleibenden Klone ("Nummer 163") kodierten für das gleiche Protein: sie wurden komplett sequenziert.

Aus einer aus Balb/c-3T3 Zellen hergestellten λZAP-cDNA Bibliothek wurden mehrere Klone identifiziert, die homolog zu p163 waren. Die Sequenzanalyse zeigt einen offenen Leserahmen; dieser wird auch in der VP16-Chimäre aus der cDNA-Bibliothek benutzt. Beide Originalklone kodieren für Aminosäure 121 bis 252 dieses Leserahmens. Die Klone p163 wurden mit β-Galaktosidase als Reportergen in Hefe näher charakterisiert:

Wie in Tabelle 1 dargestellt, interagiert das kodierte Protein in Hefe spezifisch mit p27, nicht mit anderen Proteinen wie Myc, Max, Bcl-6 oder Prothymosin-α. Tabelle 1 zeigt auch eine erste Eingrenzung der Domänen von p27, in denen die Interaktion mit p163 stattfindet. Diese Daten weisen darauf hin, daß p163 an die gleichen Domänen von p27 wie cyclinabhängige Kinasen (Russo et al., Nature 382, 325 (1996)) bindet. Dies ist ein Hinweis, daß p163 und cyclinabhängige Kinasen um die Bindung an p27 kompetieren. Zum anderen konnte die Domäne von p163, die mit p27 interagiert, auf die Aminosäure 121 bis 252 eingegrenzt werden.

Zur Bestätigung dieser Ergebnisse aus diesem Hefetestsystem wurde rekombinant exprimiertes p163 [als Fusionsprotein mit Glutathiontransferase (GST) exprimiert] an eine Säule gebunden und dann versucht, ³⁵S- markiertes p27 Protein-affinitätschromatographisch an dieser Säule zu reinigen. Durch die Verwendung des Fusionsproteins p163 mit einer Glutathiontransferase (GST) war es möglich, eine einheitliche Matrix für diese Experimente einzusetzen (sogenannte "GST-pulldowns"; Hateboer et al., Proc. Natl. Acad. Sci. USA 90, 8489 (1993)).

Für diese Untersuchungen wurde der Leserahmen aus dem Hefeklon in einen pGEX-Vektor (Pharmacia, Freiburg pGEX-2T; Best. Nr. 27-4801-01) umgesetzt, der für ein chimäres Protein mit Glutathion-S-Transferase (GST) unter der Kontrolle des IPTG-induzierbaren TAC-Promotors in E.coli kodiert (Smith and Johnson, Gene 67: 31, 1988). Chimäre Proteine wurden aus induzierten E.coli-Kulturen durch Affinitätschromatographie an Glutathion-Agarose gereinigt (Smith and Johnson, 1988) und anschließend gegen 100 mM Tris-HCI, Ph7,5, 100 mM KCl, 20 mM EDTA, 10% glycerol, 0,1 mM PMSF dialysiert. Als Kontrolle wurde Glutathion-S-Transferase nach dem gleichen Protokoll gereinigt und dialysiert: die gereinigten Proteine wurden bei -80°C gelagert.

Jeweils 10 µg der beiden Proteine wurden anschließend mit 10 µg BSA und 20 mg gequollener GST-Agarose für zwei Stunden bei 4°C inkubiert; daraufhin wurde die Agarose abzentrifugiert und zweimal mit Dialyse-Puffer gewaschen. Als Kontrolle für die Bindung der Proteine wurde ein Aliquot der Agarose direkt in Probenpuffer aufgekocht und die gebundenen Proteine nach einer SDS-Gelelektrophorese nachgewiesen. ³⁵S-Methionin-markiertes p27 wurde durch in vitro Translation (nach Angaben des Herstellers: Promega, Mannheim; TNT coupled retc. lys. systems; L 4610) hergestellt und zwei Stunden in einem Gesamtvolumen von 200 µl in Dialysepuffer mit 0.1% NP-40 bei 4°C mit beladener Agarose inkubiert. Die Agarose wurde viermal mit Dialysepuffer/0,1% NP-40 gewaschen und anschließend in SDS-Probenpuffer aufgekocht. Gebundene Proteine wurden durch SDS-Gelelektrophorese und Fluorographie nachgewiesen.

In Tabelle 2 sind die Ergebnisse dieser Versuche dargestellt. Sie belegen, daß rekombinantes, in vitro synthetisiertes, ³⁵S-markiertes p27 selektiv an eine Säule, die mit einem chimären GST-p163 Fusionsprotein beladen ist, nicht aber an eine Säule, die mit der gleichen Menge GST beladen ist, bindet. Tabelle 2 belegt auch, daß p27 aus einem Zellextrakt ineffizient an GST-p163 bindet; in diesem Extrakt ist p27 an Komplexe cyclinabhängiger Kinasen gebunden. Eine kurze Hitzebehandlung setzt p27 aus diesen Komplexen frei und erlaubt dann die Bindung an p163. Dies belegt, daß p163 und cyclinabhängige Kinasen um die Bindung an p27 kompetieren.
Die erfindungsgemäße Nukleotidsequenz, welche für das murine Protein p163 kodiert, ist in Tabelle 3 dargestellt. Diese Sequenz hat ein Homolog in der Datenbank; es handelt sich um das Nukleoprotein (Nup)2 Gen der Hefe. Nup2 ist ein Protein der Kernmembran, das an der Bildung von Kernprotein und am Transport von Proteinen in und aus dem Kern beteiligt ist. Nup2 ist in der Hefe wohl vor allem am Export beteiligt.

Mehrere funktionelle Domänen sind konserviert, obwohl insgesamt die Homologie nicht hoch ist (Tabelle 4). So ist die Bindestelle für ein regulatorisches Protein (Ran, ein GTP-bindendes Protein), das den Kerntransport reguliert, zwischen Ran binding protein (RBP-1), NuP-2 und p163 konserviert (Tabelle 5), wie auch kurze Pentapeptidsequenzen, denen strukturelle Funktion zugesprochen wird (Tabelle 6).

Um nachzuweisen, daß es sich bei p163 zweifellos um ein Nukleoporin handelt, wurde ein polyklonales Antiserum gegen das Protein [Histidin-p163 (aa 121-252)] erzeugt und affinitätsgereinigt; dieses Antiserum erkennt fraglos ein Nukleoporin, was in der Immunfluoreszens an der typischen Kernporenfärbung ("dots" auf der Oberfläche und die Kernperipherie ist gefärbt) erkennbar ist.

Zum Nachweis einer intrazellulären Assoziation zwischen p163 oder Nup2 und p27 wurden beide Proteine in HeLa-Zellen transient mit CMV-Expressionsvektoren exprimiert, 48 Stunden später die Zellen lysiert und auf eine mögliche Interaktion beider Proteine durch Immunpräzipitation/Western Blots untersucht (Peukert et al., EMBO J. 16, 5672 (1997)). Die Assoziation zwischen p27 und Nup2 wurde mit je einem polyklonalen Antikörper gegen Nup2 und gegen murines p27 nachgewiesen. Das Protein p27 wurde nur dann gut präzipitiert, wenn p163 exprimiert wurde (siehe Tabelle 7). Dies ist ein Nachweis, daß Nup2 und p27 in HeLa-Zellen in vivo miteinander assoziieren können.

Durch Sequenzvergleich und Datensuche mit humanen EST's wurde die Nukleinsäuresequenz für das humane p163 Protein identifiziert. Für die Ran Bindedomäne liegt sie in der Sequenz AA161285 in den Positionen 173 ≥ 575, für die p27 Bindedomäne in der Sequenz R62312 in den Positionen 318 ≥ 486, für andere Teilabschnitte von p163 in der Sequenz THC199124 in den Positionen 348 ≥ 489.

Um genauer zu definieren, mit welchen Aminosäuren von p27 die Interaktion von Nup2-p163 stattfindet, wurden in Hefe nach Mutanten gesucht, die nicht mehr mit p163 interagieren. Dazu wurde die cDNA, kodierend für p27 einer fehlerinduzierenden PCR-Reaktion (PCR-Bedingungen gemäß "PCR-technology, Principles and Applications for DNA amplifications"; H.A. Erlich, Stockton press, NY 1989) unterzogen und die resultierenden Klone, die zufällige Fehler enthalten, auf eine Interaktion mit Nup2 und, als Kontrolle, mit Cyclin D1 getestet. Die (vom Standard abweichenden) Bedingungen dieser PCR waren: 40 Zyklen in Gegenwart von 1U GIBCO-Taq Polymerase und von 0.1 mM MnCl₂.

Das erhaltene cDNA-Fragment wurde mit einem pGBT10-Vektor, der mit BamHI und EcoRI linearisiert und gereinigt worden war, in den Hefestamm HF7c-p163/Nup2 co-transformiert. Transformierte Kolonien wurden durch Selektion aus -Leu-Trp Mangelmedium selektioniert. 960 einzelne Klone wurden isoliert und auf selektives Medium (-Leu-His-Trp) plattiert. Mit Klonen, die auf diesem selektiven Medium nicht wuchsen, wurde ein β-Galaktosidase Test als zweiter Interaktionstest durchgeführt. Negative Klone wurden identifiziert und aus ihnen das pGBT10-p27 Plasmid isoliert. Die gewonnenen Plasmide wurden erstens mit einem Hefe-Expressionsplasmid retransformiert, der p163/Nup2 als Chimäre mit einer transaktivierenden Domäne exprimiert, und zweitens als Kontrolle mit einem Plasmid, das Cyclin D1 als Chimäre mit einer transaktivierenden Domäne exprimiert. Drei Klone wurden erhalten, die selektiv nicht mehr mit Nup2, wohl aber mit Cyclin D1 interagieren. Diese Plasmide wurden zusammen mit einigen Kontrollen sequenziert.

Eine Auswahl der erhaltenen Phänotype ist in Tabelle 13 zusammengefaßt. In diesen Versuchen zeigt sich eine Interaktion als Wachstum in der Abwesenheit von Histidin(-Trp-Leu-His, "selektiv"), während die Kontrolle (-Trp-Leu; "nicht selektiv") zeigt, daß beide Proteine in Hefe exprimiert wurden. Aus 1000 Klonen wurden 3 gefunden, die spezifisch nicht mehr mit p163-Nup2, wohl aber mit Cyclin D1 interagieren. Die Sequenzen sind in Tabelle 14 gezeigt. Alle drei Klone, nicht aber eine Reihe von Kontrollklonen, tragen die gleiche Mutation in der Aminosäure Arginin 90 (mutiert zu Glycin). Die Sequenzen zeigen auch, daß die Klone unabhängig entstanden sind, denn sie tragen weitere, unterschiedliche Mutationen. Damit ist Arginin 90 eindeutig als zentrale Aminosäure in der Wechselwirkung von p27 mit Nup2 definiert. Die Mutanten stehen für eine Validierung der biologischen Relevanz dieser Interaktion zur Verfügung.

### 2) Nukleotidsequenzen für dominant negativ wirkende Analoga des p163 Proteins

Durch die oben beschriebenen Funktionsanalysen sind zwei Domänen des p163 Proteins bekannt, welche für seine Funktion entscheidend sind.

Zum einen ist dies die Domäne (Aminosäuren 121-252) zur Bindung an p27. Zum anderen ist dies die Domäne (Aminosäuren 307-467) zur Bindung an Ran, einem GTP-bindenden Protein, das Transportfunktionen reguliert.

Gegenstand der Erfindung sind somit Nukleinsäuresequenzen für das Protein p163 oder für Teile dieses Proteins, wobei diese Nukleinsäuresequenzen Mutationen in der die p27 bindenden und/oder der die Ran bindenden Domäne aufweisen, so daß die Bindung des exprimierten mutierten Proteins entweder an p27 oder an Ran drastisch verringert ist. Eingeführt in eine Zelle inhibieren derartige Analoga von p163 das funktionsfähige zelleigene p163. Hierdurch entsteht freies p27, was zur Inhibition der Zellteilung führt. Beispiele für derartige dominant negative Mutanten von p163 sind in den Tabellen 8 und 9 dargestellt. Tabelle 8 zeigt p163 mit einer Deletion der p27 bindenden Domäne, Tabelle 9 zeigt p163 ohne die Ran-bindende Domäne.

Gegenstand der Erfindung ist somit die Verwendung von dominant negativ-interferierenden p163 Analoga in einer Zelle mit dem Ziel, die Proliferation dieser Zelle zu inhibieren.

### 3) Nukleotidsequenzen für Proteine oder Peptide, die an die Bindedomäne von p163 binden

Durch die vorgelegten Untersuchungen konnte die p27 Bindedomäne des p163 Proteins auf die Aminosäuren 121-252 und die Ran Bindedomäne auf die Aminosäuren 307-467 festgelegt werden.

Ein weiteres Beispiel sind Antikörper oder Antikörperfragmente wie F(ab)₂, Fab, Fv oder s.c. Fv, welche an p163 binden, im besonderen an die p27 Bindedomäne oder an die Ran Bindestelle von p163. Derartige Antikörper können beispielsweise durch Immunisierung von Tieren mit p163 oder der p27-bindenen Domäne oder Ran-bindenden Domäne von p163 gewonnen werden. Diese Domänen sind in Tabelle 5 (Ran-bindende Domäne) oder in Tabelle 10 (p27-bindende Domäne) dargestellt.

### 4) Nukleotidsequenzen für p163 zur Stimulation der Zellteilung

Die Einführung der Nukleotidsequenz für p163 oder des Proteins p163 in eine Zelle führt zur Inhibition des intrazellulären p27. Hierdurch werden Cyclin E/cdk2 Komplexe (die durch p27 inhibiert werden) frei, die die Zellteilung initiieren.

### 5) Nukleotidsequenzen zur Hemmung der Transkription und/oder Translation des p163 Proteins

Die Kenntnis der Nukleinsäuresequenz des p163 Proteins bietet die Möglichkeit, Oligonukleotide herzustellen mit Hilfe derer die Transkription oder Translation von p163 spezifisch inhibiert werden kann. Zu diesen Antisense-Nukleinsäuren gehören Oligonukleotide, antisense (Triplex-)DNA, Ribozyme oder antisense RNA. Die Methode der Herstellung von Triplex-DNA-Oligonukleotiden ist ausführlich von Frank-Kamenetskii und Mirkin, Ann. Rev. Biochem 64, 65 (1995) und die Methode der antisense RNA-Oligonukleotidherstellung von Neckers et al., Crit. Rev. Oncogen. 3, 175 (1992); Carter und Lemoine, Br. J. Cancer 67, 869 (1993); Mukhdopadhyay und Roth, Crit. Rev. Oncogen. 7, 151 (1996) und Mercola und Cohen, Cancer Gene Ther. 2, 47 (1995) beschrieben worden.

Bevorzugt im Sinne der Erfindung sind Triplex-DNA oder antisense RNA-Oligonukleotide zu verwenden, welche mit Nukleotidsequenzen des p163 hybridisieren, welche für Teilbereiche der Bindedomäne (Gesamtbereich Aminosäure 121-252) für das p27 Protein oder für Teilbereiche der Bindedomäne (Gesamtbereich Aminosäure 307-467) für das Ran Protein kodieren.

Zu den Nukleotidsequenzen, welche die Transkription und/oder die Translation von p163 hemmen, gehören desweiteren Ribozyme spezifisch für Nukleotidsequenzbereiche des p163 Proteins.

Die Methode der Herstellung von Ribozymen wurde ausführlich von Burke, Nucl. Acids Mol. Biol. 8, 105 (1994), Christoffersen und Marr, J. Med. Chem 38, 2023 (1995) und Scott et al., Science 274, 2065 (1996) beschrieben. Bevorzugt im Sinne der Erfindung sind Ribozyme, die mit Nukleotidsequenzen des p163 hybridisieren, die im Bereich der Nukleotidsequenz für die Bindedomäne (Aminosäuren 121-252) für das p27 Protein oder für die Bindedomäne (Aminosäure 307-467) für das Ran Protein liegen.

Beispiele für durch Ribozyme spaltbare Nukleotidsequenzen des p163 Proteins sind in Tabelle 11 aufgeführt.

Triplex-DNA, antisense RNA oder Ribozyme werden als Oligonukleotide [hergestellt und gegen den Abbau durch DNAsen bzw. RNAsen geschützt nach den dem Fachmann bekannten Methoden (siehe die oben zitierte Literatur)] entweder in vitro zu den Zielzellen gegeben oder in vivo durch Injektion oder lokale Applikation verabreicht.

Gegenstand der Erfindung ist jedoch auch die Einführung von Nukleinsäurekonstrukten in eine Zelle mit dem Ziel, daß sich in der Zelle durch Transkription die antisense RNS oder Ribozyme entsprechend den erfindungsgemäßen Oligonukleotide entstehen, um die Proliferation dieser Zelle zu inhibieren. Die Einführung in die Zelle kann in vitro aber auch in vivo erfolgen.

Das Nukleinsäurekonstrukt wird in die Zelle als nackte DNA oder mit Hilfe eines nichtviralen Vektors oder mit Hilfe und eingefügt in einen viralen Vektor nach der dem Fachmann bekannten Verfahren eingebracht.

### 6) Testsysteme zur Auffindung von Inhibitoren der Funktion von p163

Die Entdeckung des p163 Proteins und seiner Wechselwirkung mit dem p27 Protein ermöglicht die Suche nach Inhibitoren dieser Wechselwirkung. Hierfür sind Testsysteme vonnöten, bei welchen die Bindung zwischen dem p27 Protein und dem p163 Protein gehemmt wird und diese Hemmung durch einen Indikator angezeigt wird.

Zahlreiche Methoden sind bekannt. Ein Beispiel für diese Methode ist der "Two Hybrid Screen Assay" (siehe hierzu Abschnitt C.1. und Tabelle 1).

Alternativ kann jedoch auch beispielsweise ein Affinitätssystem zum Screening verwendet werden, bei welchem p163 [bevorzugt die p27 bindenden Domäne (Aminosäure 121-252)] an eine feste Phase gebunden ist, mit der Prüfsubstanz inkubiert wird und die Bindung der Prüfsubstanz durch Hemmung der Bindung eines markierten Bindungspartners für die p27 bindende Domäne des p163 Proteins ermittelt wird. Dieser markierte Bindungspartner kann p27 sein oder ein an die p27 bindende Domäne des p163 Proteins spezifisch bindender Antikörper. In gleicher Weise wie für Inhibitoren der Bindung zwischen p163 und p27 können auch Testsysteme für Inhibitoren der Bindung zwischen p163 und Ran aufgebaut und für das Screening genutzt werden. Gegenstand der Erfindung ist somit die Verwendung von Nukleinsäuresequenzen kodierend für p163 oder Teilproteinen von p163 oder die Verwendung des p163 Proteins oder Teilproteinen hiervon für die Suche nach Inhibitoren für p163.

### 7) Verwendung der Nukleinsäuresequenz für p163 oder der Proteinsequenz p163 zur Diagnose des Proliferationsstadiums einer Zelle oder eines Krankheitszustandes

Wie in der Einführung Abschnitt A) dargestellt, zeichnen sich eine Reihe von Tumoren durch eine erhöhte intrazelluläre Konzentration von p27 aus. Aufgrund der Tatsache, daß diese Tumoren proliferieren ist anzunehmen, daß die Funktion dieses erhöhten p27 gehemmt ist. Entsprechend dieser Erfindung ist p163 der spezifische Inhibitor von p27. Der Nachweis dieses Inhibitors in einer Zelle erlaubt eine Aussage über den Proliferationszustand der Zelle. Diese Aussage kann z.B. zur Beurteilung der Malignität einer Tumorzelle oder eines Tumorgewebes von großer Wichtigkeit sein. Durch Zelltod wird das Protein p163 freigesetzt. Der Nachweis von p163 in einer Körperflüssigkeit erlaubt also die Aussage über proliferative und/oder mit Zelltod einhergehende, beispielsweise entzündliche Prozesse in einem Körper. Der Nachweis des Proteins p163 kann durch spezifische Bindung an markierte Substanzen erfolgen. Zu diesen spezifisch bindenden Substanzen gehören das p27 Protein, das Ran Protein und Antikörper, beispielsweise erzeugt durch Immunisierung von Tieren mit dem Protein p163 oder mit Teilsequenzen dieses Proteins.

Der Nachweis der p163-mRNA in einer Zelle oder einem Gewebe kann jedoch auch erfolgen durch Hybridisierung der das Protein p163 kodierenden Nukleinsäuresequenz mit der korrespondierenden mRNA. Eine Erhöhung der Sensitivität dieses Nachweises ist durch die dem Fachmann geläufige "Polymerase Chain Reaction" (PCR)-Technologie möglich, bei welcher wenige Kopien einer nachzuweisenden Nukleinsäuresequenz mit Hilfe sogenannter Primer-Paare vermehrt werden können.

Beispiele für Primer-Paare zur Vermehrung und zum Nachweis der Nukleinsäuresequenz kodierend für p163 sind in den Tabellen 12a, b und c dargestellt.

Der Nachweis der RNA kodierend für p163 ist jedoch auch möglich beispielsweise mit der von Gussoni et al., Nature Biotechnol. 14, 1012 (1996) publizierten Fluoreszenz in situ Hybridisierung.

Gegenstand der Erfindung ist somit der Nachweis einer Nukleinsäuresequenz kodierend für p163 oder des Proteins p163 für die Ermittlung des Proliferationszustandes einer Zelle oder eines Gewebes oder dem Nachweis von proliferativen oder mit Zelltod einhergehenden Veränderungen in einem lebenden Säuger.

Die Gegenstände der vorliegenden Erfindung lassen sich wie folgt zusammenfassen:
1) Ein Protein, welches p27 inhibiert und hierdurch die durch das Protein p27 bewirkte Hemmung der Zellproliferation aufhebt.
2) Protein gemäß Satz 1, dadurch gekennzeichnet, daß es die Aminosäuresequenz von P163 gemäß Tabelle 6 [SEQ ID NO.: 16] oder Teile davon enthält.
3) Protein gemäß Satz 2, dadurch gekennzeichnet, daß die Teile der Aminosäuresequenz von p 163 ausgewählt werden aus der Gruppe enthaltend Peptide mit den Aminosäuresequenzen von Position 1 bis 24 [SEQ ID No.: 2], von Position 137 bis 196 [SEQ ID NO.: 4], von Position 215 bis 265 [SEQ ID NO.: 6], von Position 239 bis 272 [SEQ ID NO.: 8], von Position 399 bis 438 [SEQ ID NO.: 10] und von Position 307 bis 469 [SEQ ID NO.: 12] bezogen auf die Numerierung gemäß Tabelle 6.
4) Protein, welches durch Deletion funktioneller Domänen von dem Protein gemäß Satz 2 abgeleitet werden kann, dadurch gekennzeichnet, daß von der [SEQ ID NO. 16] der Tabelle 6 die p27-bindende Domäne oder die Ran-bindende Domäne deletiert ist.
5) Protein gemaß Satz 4, dadurch gekennzeichnet, daß nach Deletion der p27-bindenden Domäne ein Protein der Aminosäuresequenz gemäß Tabelle 8 [SEQ ID NO.: 18] und bei Deletion der Ran-bindenden Domäne ein Protein der Aminosäuresequenz gemäß Tabelle 9 [SEQ ID NO.: 19] vorliegt.
6) Protein, welches durch Deletion aller Aminosäuresequenzen außer dem Bereich der p27-bindenden Domäne von dem Protein gemäß Satz 2 abgeleitet werden kann.
7) Protein gemäß Satz 6, dadurch gekennzeichnet, daß es die Aminosäuresequenz gemäß Tabelle 10 [SEQ ID NO.: 20] enthält.
8) Protein gemäß einem oder mehreren der Sätze 1 bis 7, dadurch gekennzeichnet, daß es sich um das entsprechende homologe Protein oder die entsprechenden Teile davon, einer anderen Säugetierspezies oder des Menschen handelt.
9) Protein gemäß Satz 8, dadurch gekennzeichnet, daß es die Aminosäuresequenz des menschlichen Homologs des p163 enthält.
10) DNA, kodierend für ein Protein gemäß einem oder mehreren der Sätze 1 bis 9.
11) DNA gemäß Satz 10, dadurch gekennzeichnet, daß sie
   (a) die Nukleotidsequenz gemäß Tabelle 3 [SEQ ID NO.: 1] oder Teile davon enthält,
   (b) unter stringenten Bedingungen mit der unter (a) genannten Sequenz hybridisiert, oder
   (c) gegenüber der unter (a) genannten Sequenz entsprechend der Degeneriertheit des genetischen Codes verändert ist, jedoch für die gleiche Aminosäuresequenz kodiert.
12) DNA gemäß Satz 11 zur Verwendung als PCR-Primer, dadurch gekennzeichnet, daß die Nukleotidsequenzen dieser PCR-Primer ausgewählt werden aus der Gruppe enthaltend die [SEQ ID NO: 21] bis [SEQ ID NO.: 30] der Tabelle 12a, die [SEQ ID NO.: 31] und [SEQ ID NO.: 32] der Tabelle 12b und die [SEQ ID NO.: 33] bis [SEQ ID NO.: 40] der Tabelle 12c.
13) Verwendung der DNA nach einem der Sätze 10 oder 11 zum Nachweis und/oder Quantifizierung der p163 mRNA in Zellen und/oder Geweben.
14) Verwendung gemäß Satz 13, dadurch gekennzeichnet, daß der Nachweis und/oder die Quantifizierung durch Northern Blotting erreicht wird.
15) Verwendung gemäß Satz 13, dadurch gekennzeichnet, daß der Nachweis und/oder die Quantifizierung durch PCR erreicht wird.
16) Verwendung gemäß Satz 15, dadurch gekennzeichnet, daß die PCR-Primer gemäß Anspruch 12 angewandt werden.
17) Verwendung gemäß Satz 13, dadurch gekennzeichnet, daß der Nachweis und/oder die Quantifizierung durch Fluoreszenz in situ Hybridisierung erreicht wird.
18) Verwendung des Proteins gemäß einem oder mehreren der Sätze 1 bis 9 zur Herstellung von Antikörpern, die an dieses Protein binden.
19) Antikörper und Spaltprodukte dieser Antikörper, welche an die Bindung für das p27-Protein des Proteins gemäß einem oder mehreren der Sätze 1 bis 9 binden.
20) Antikörper und Spaltprodukte dieser Antikörper, die an die Bindedomäne für das Ran-Protein des Proteins gemäß einem oder mehreren der Sätze 1 bis 9 binden.
21) Verwendung von Antikörpern gemäß der Sätze 19 oder 20 zum Nachweis des Proteins gemäß einem oder mehreren der Ansprüche 1 oder 2 in Zellen, Geweben oder Körperflüssigkeiten.
22) Antisense-Nukleinsäure, welche komplementär zu Teilbereichen der DNA gemäß einem der Sätze 10 oder 11, wobei diese Teilbereiche im Bereich der für die Aminosäuren 121 bis 467 kodierenden Nukleotide liegen.
23) Antisense-Nukleinsäure gemäß Satz 22, dadurch gekennzeichnet, daß die Antisense-Nukleinsäure ausgewählt wird aus der Gruppe enthaltend triple-DNA, synthetische Oligonukleotide, antisense RNA und Ribozyme.
24) Verwendung der Antisense-Nukleinsäure nach den Sätzen 22 oder 23 zur Hemmung der Proliferation einer Zelle dadurch charakterisiert, daß diese Zelle in vitro oder in vivo mit den Antisense-Nukleinsäure in Kontakt gebracht wird.
25) Nukleinsäurekonstrukt kodierend für Antisense-Nukleinsäure nach Satz 22 oder 23 zur Hemmung der Proliferation einer Zelle dadurch charakterisiert, daß eine DNA kodierend für die Antisense-Nukleinsäuresequenz mit mindestens einer Aktivierungssequenz verbunden wird und als nackte DNA, eingefügt in einen nichtviralen Vektor oder eingefügt in einen viralen Vektor, in die Zielzelle eingeführt wird.
26) Verwendung von Proteinen gemäß einem oder mehreren der Sätze 1 bis 9, dadurch gekennzeichnet, daß es zur Suche nach Inhibitoren der Wechselwirkung zwischen besagtem Protein und zellulären Bindungspartnern benutzt wird.
27) Verwendung gemäß Satz 26, dadurch gekennzeichnet, daß das "Two-Hybrid-System" verwendet wird.
28) Verwendung gemäß Satz 26, dadurch gekennzeichnet, daß ein Affinitätssystem benutzt wird, bei dem p163 oder die p27-bindende Domäne davon an eine feste Phase gebunden wird, diese feste Phase dann mit einer Prüfsubstanz inkubiert wird und schließlich mittels eines Proteins gemäß der Sätze 1 bis 9 die Hemmung der Bindung des Bindungspartners mit dem genannten Protein ermittelt wird.
29) Verwendung gemäß Satz 28, dadurch gekennzeichnet, daß die Bindungspartner ausgewählt werden aus der Gruppe enthaltend p27 und Ran.
30) Verwendung eines Proteins gemäß einem oder mehreren der Sätze 3 bis 9 als Inhibitor der Funktion eines Proteins gemäß einem oder mehreren der Sätze 1, 2, 8 und 9.
31) Verwendung eines Antikörpers nach Satz 19 oder 20 als Inhibitor der Funktion eines Proteins gemäß einem oder mehreren der Ansprüche 1, 2, 8 und 9.
32) Nukleinsäurekonstrukt enthaltend eine DNA gemäß Satz 10 oder 11 operativ gekoppelt an eine Aktivierungssequenz, die die Expression eines Proteins gemäß einem oder mehreren der Sätze 1 bis 9 in einer Zelle ermöglicht.
33) Verwendung eines Nukleinsäurekonstruktes gemäß Satz 32 zur Stimulation der Zellproliferation.

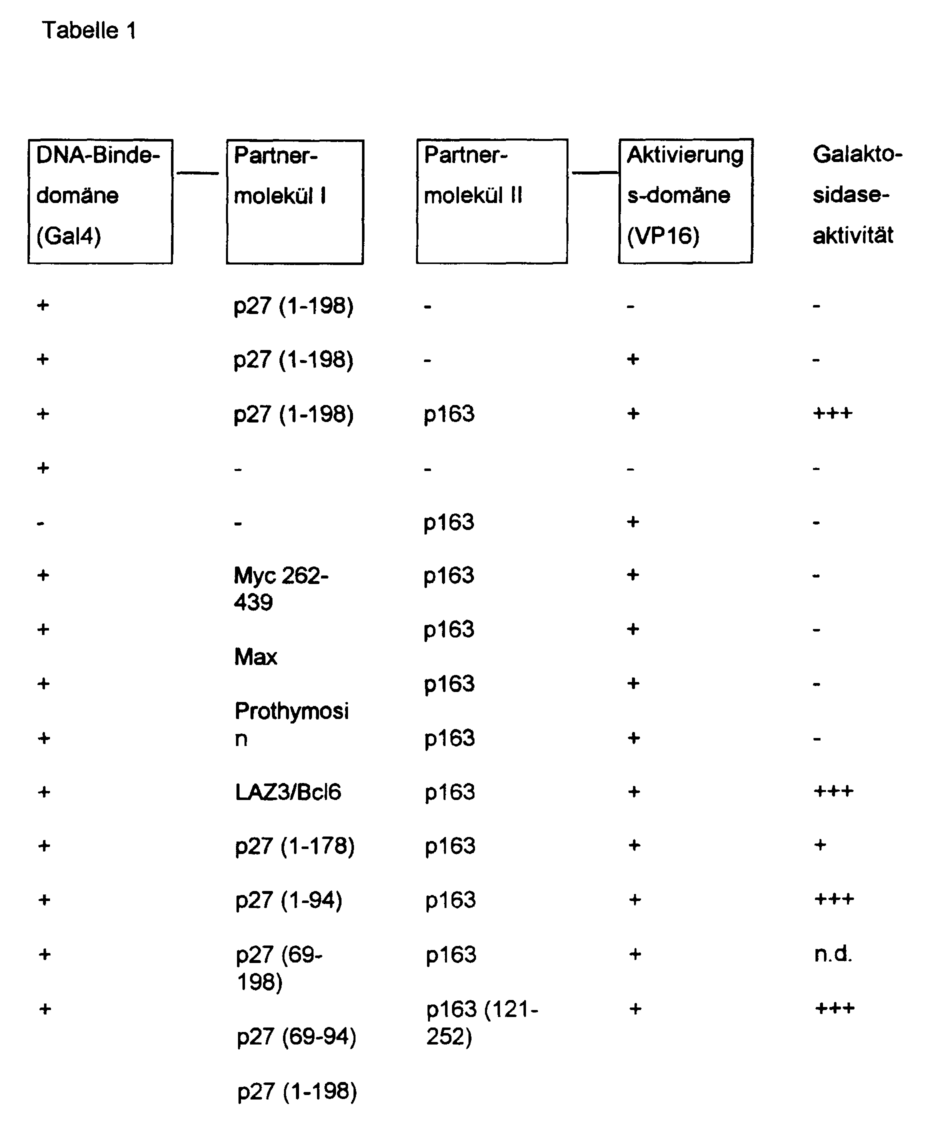

**Tabelle 2**

| Säulenauftrag | Bindung an Affinitätschromatographiesäule beschichtet mit | |
|---|---|---|
| | Fusionsprotein Glutathiontransferase - p163 | Glutathiontransferase |
| rekombinantes (³⁵S-markiertes) p27 | +++ | + |
| Zellextrakt (RAT1-Myc ER), enthaltend p27 | (+) | - |
| Zellextrakt, RAT1-Myc ER), enthaltend p27 (erhitzt 95°C, 2 min) | +++ | - |
| ckd-2 (Zellextrakt) | - | - |
| cdk-4 (Zellextrakt) | - | - |

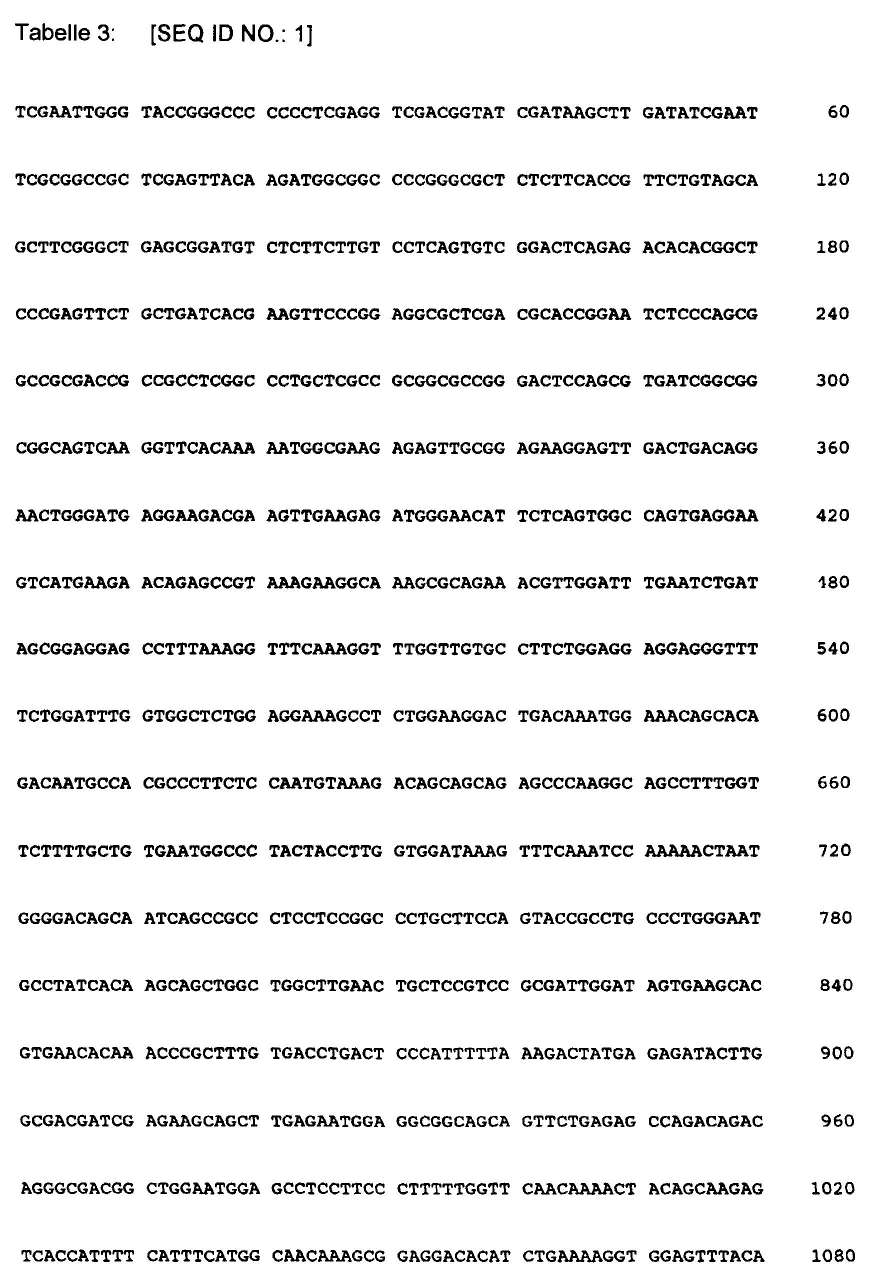

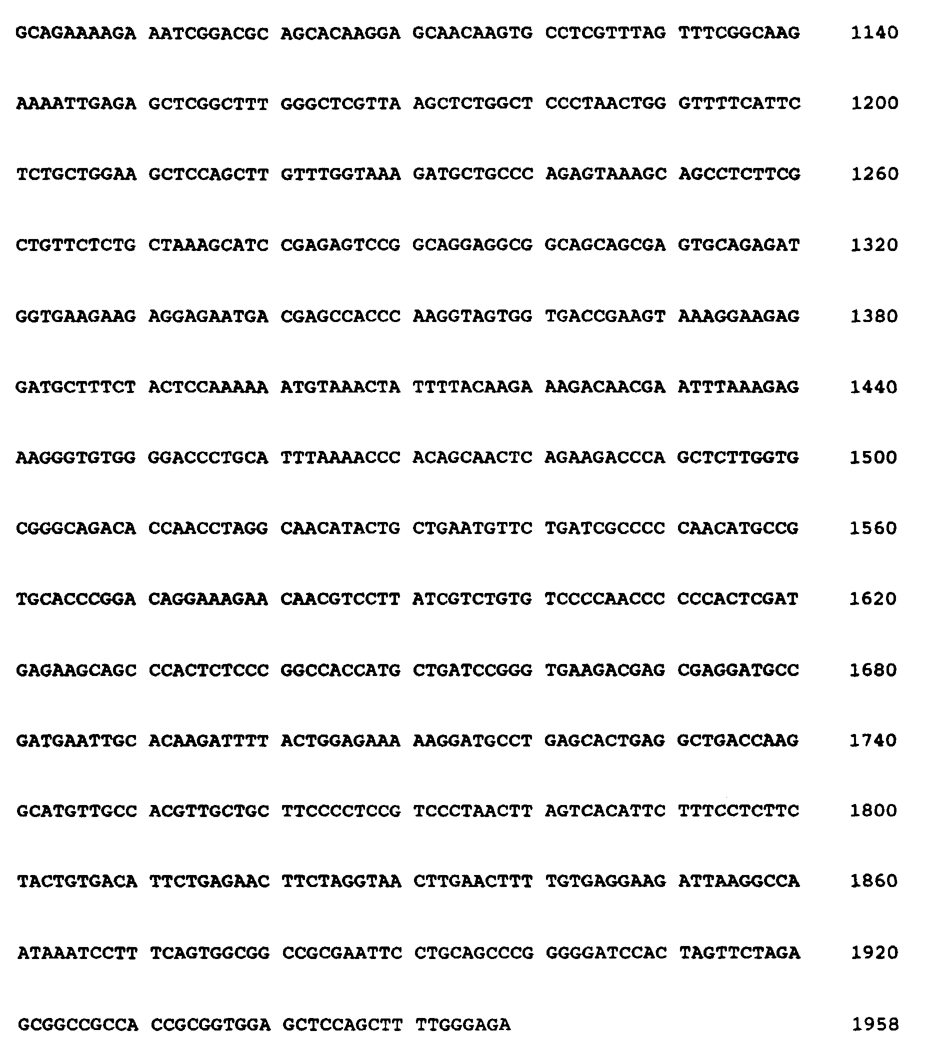

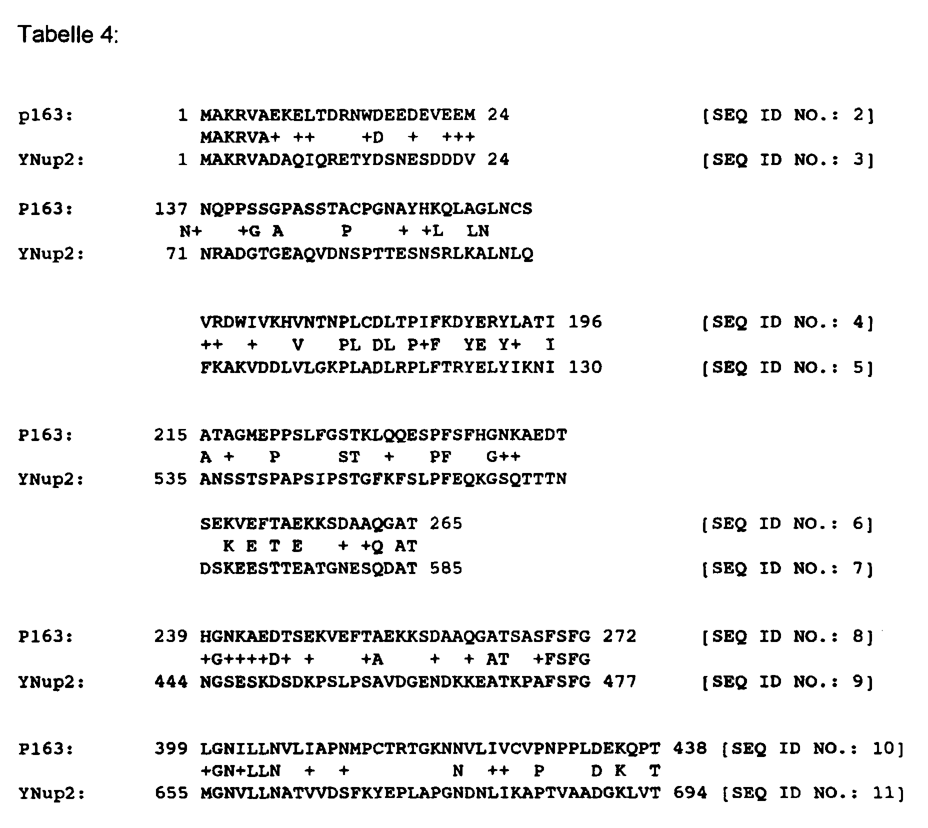

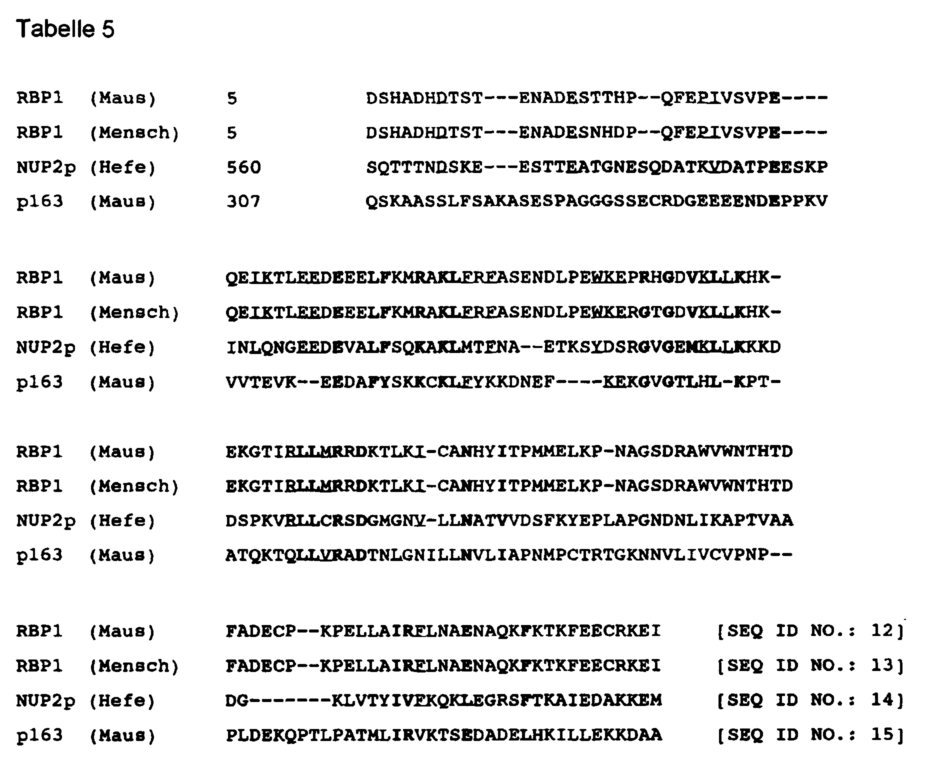

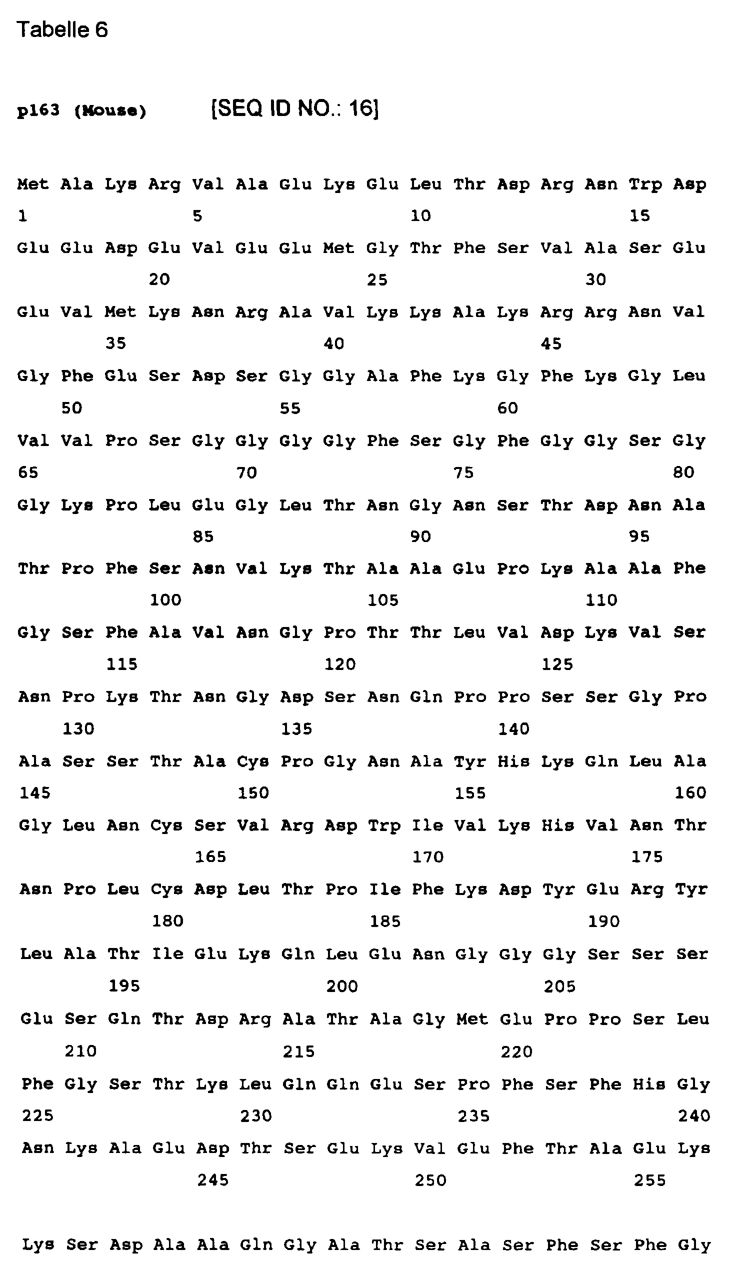

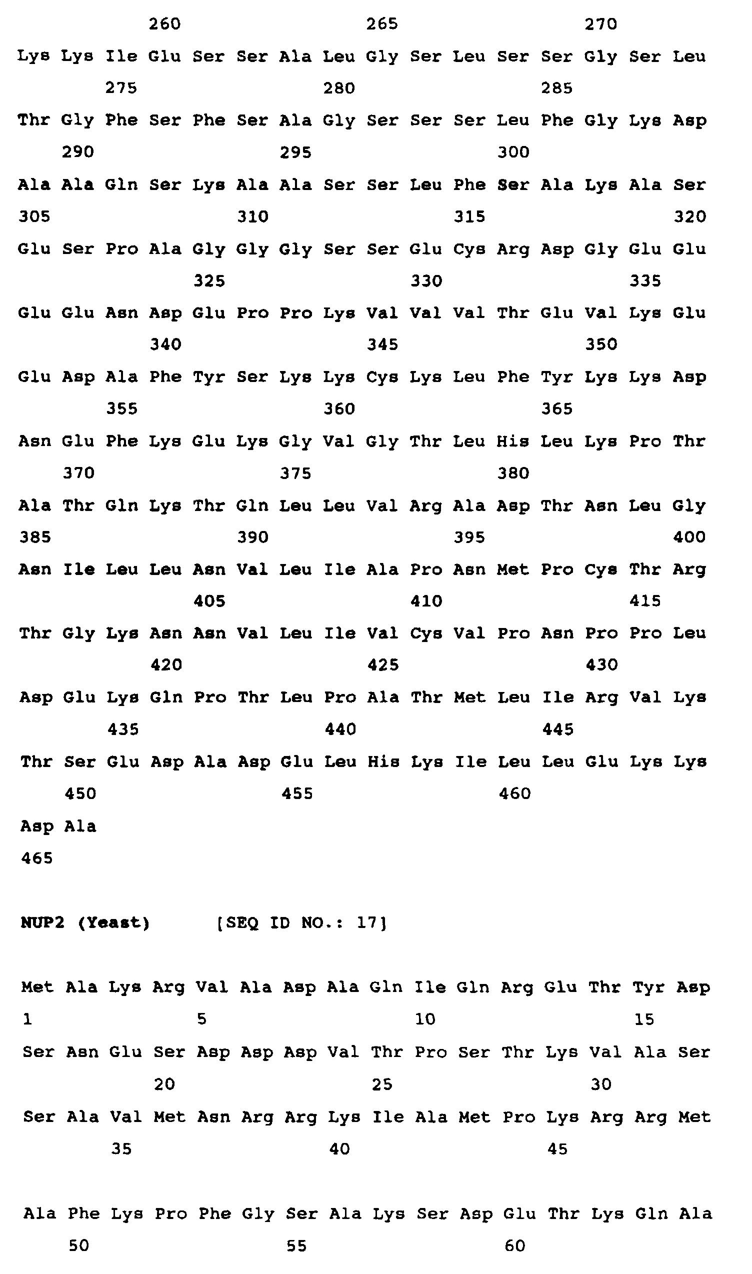

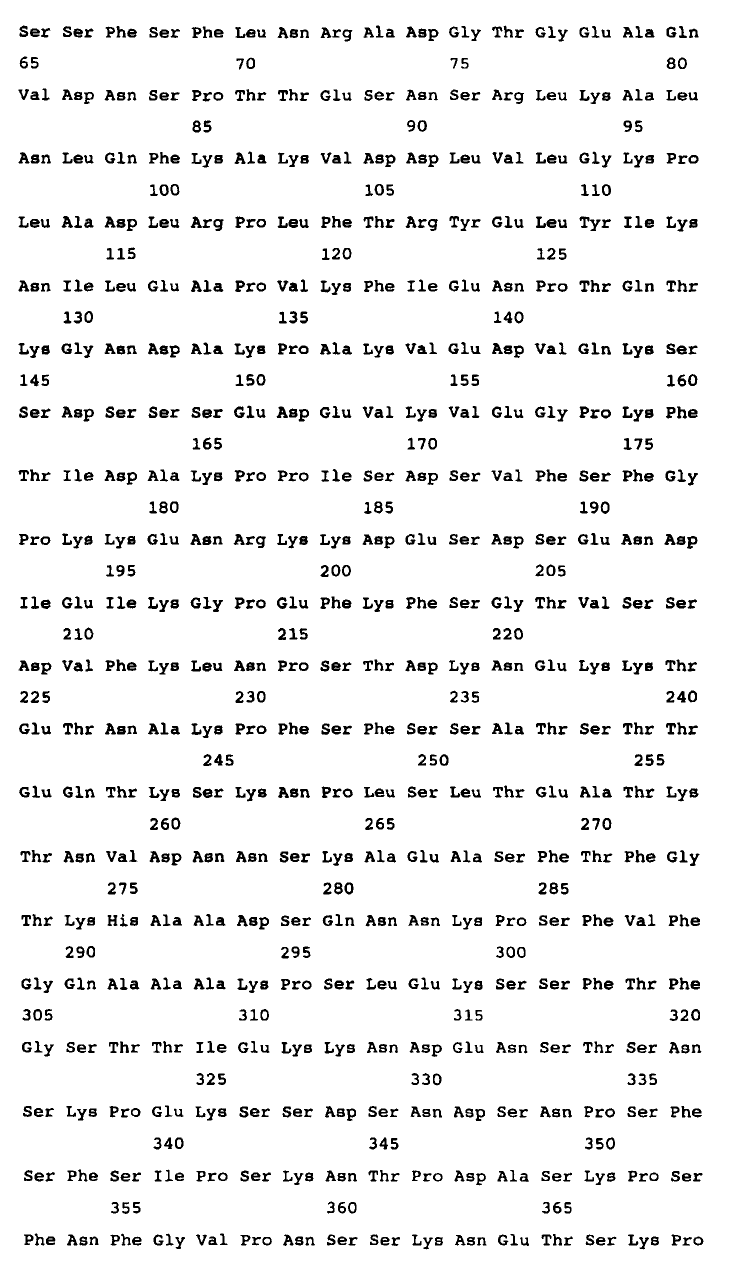

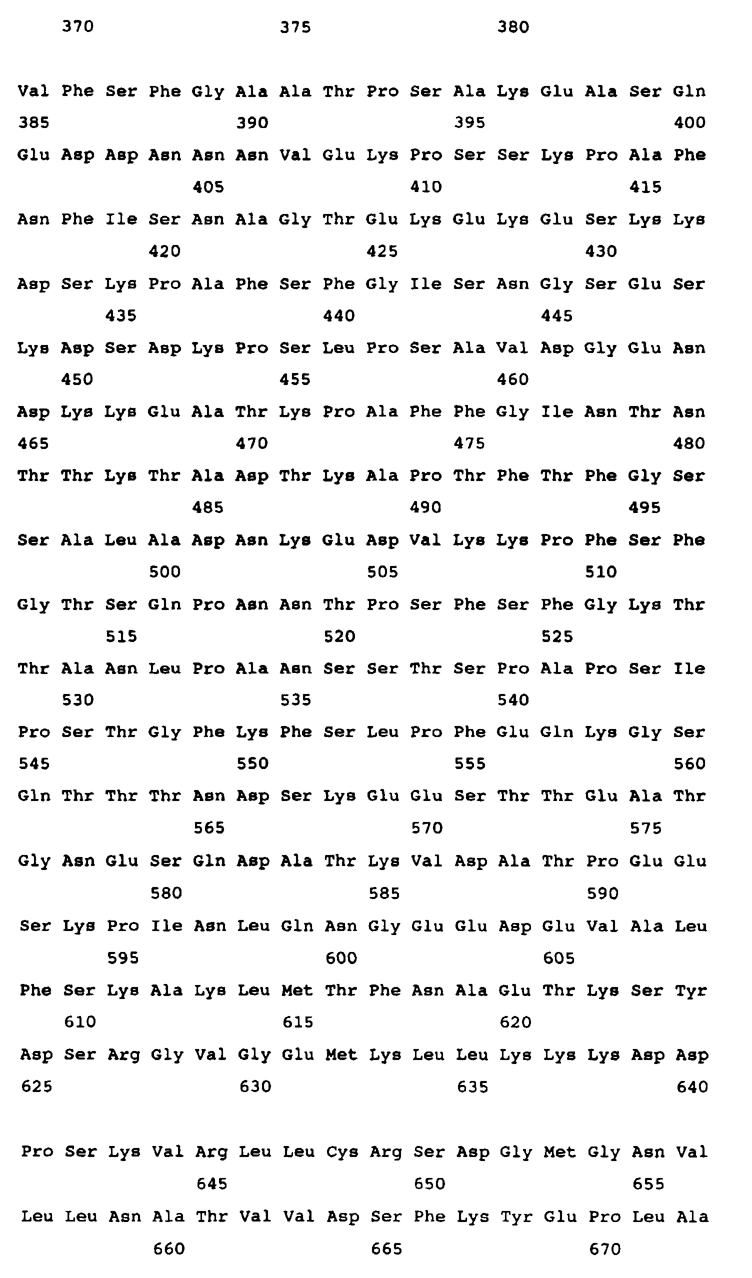

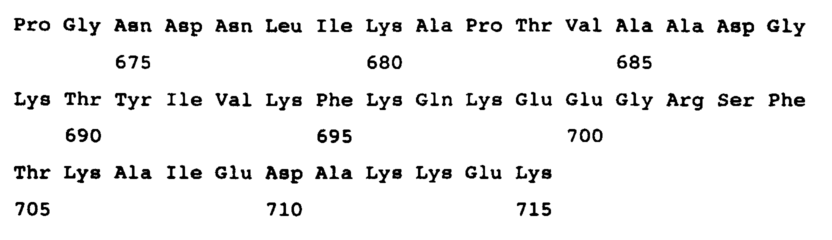

**Tabelle 7**

| Nachweis von Assoziationen zwischen p27 und p163 in HeLa-Zellen | | | | |
|---|---|---|---|---|
| | Präzipitatoin des Gesamtproteins von HeLa-Zellen transfiziert mit | | | |
| Präzipitation mit | CMV-p27 | CMV-p27 + CMV-Nap | - | - |
| | | | CMV-Nap2 | - |
| Antikörper spezifisch für Nap2 | - | + | + | - |
| Antikörper spezifisch für p27 | + | ++ | - | - |

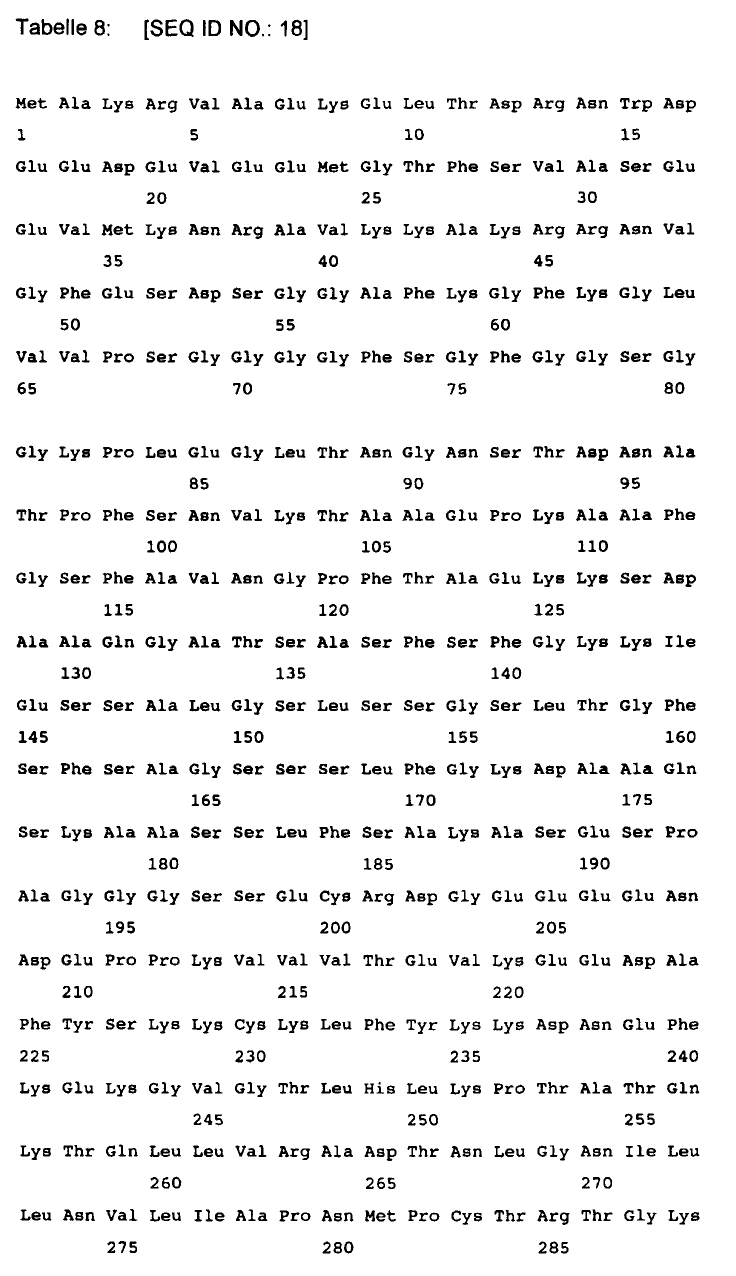

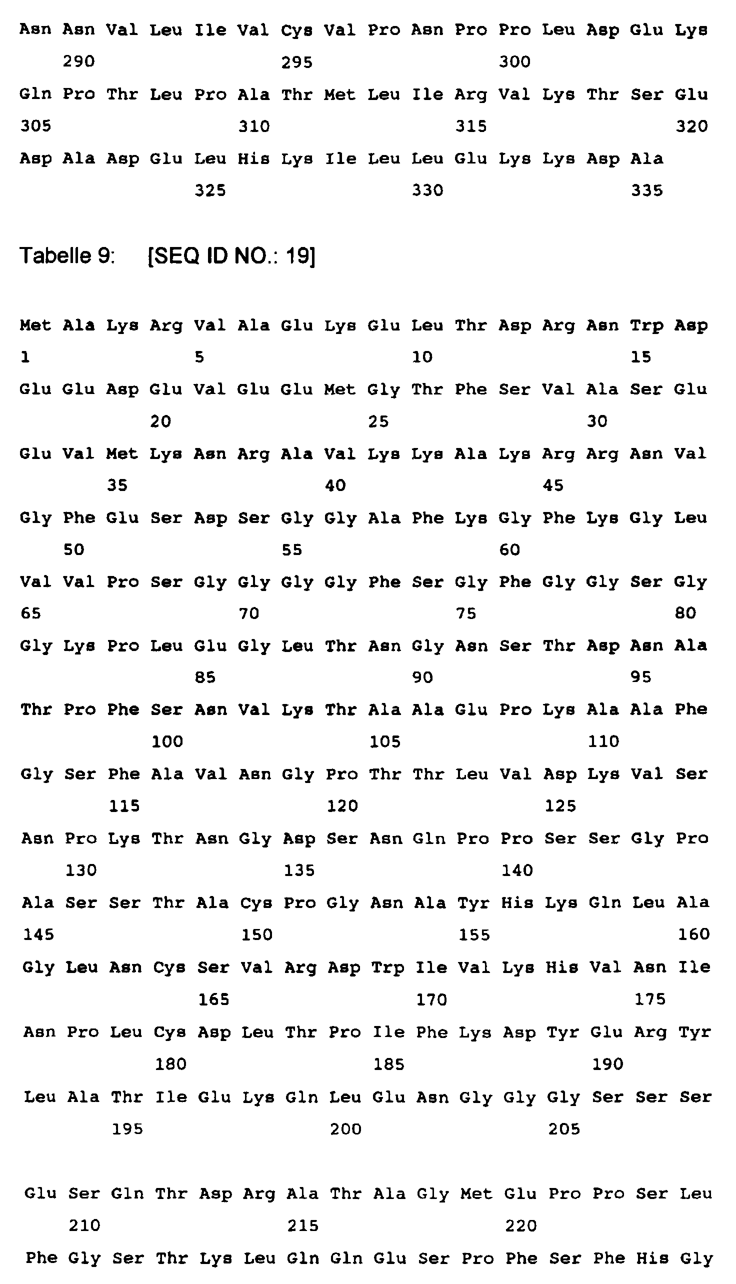

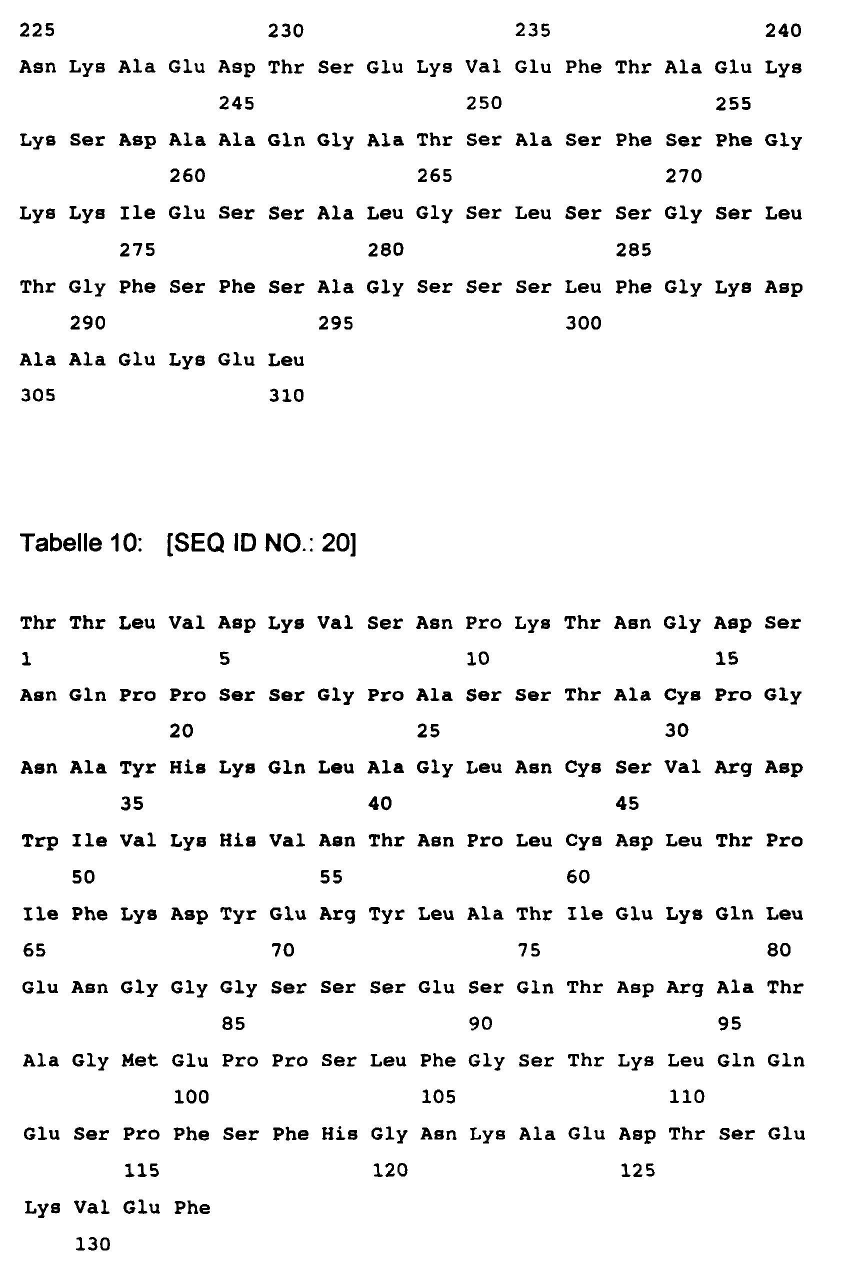

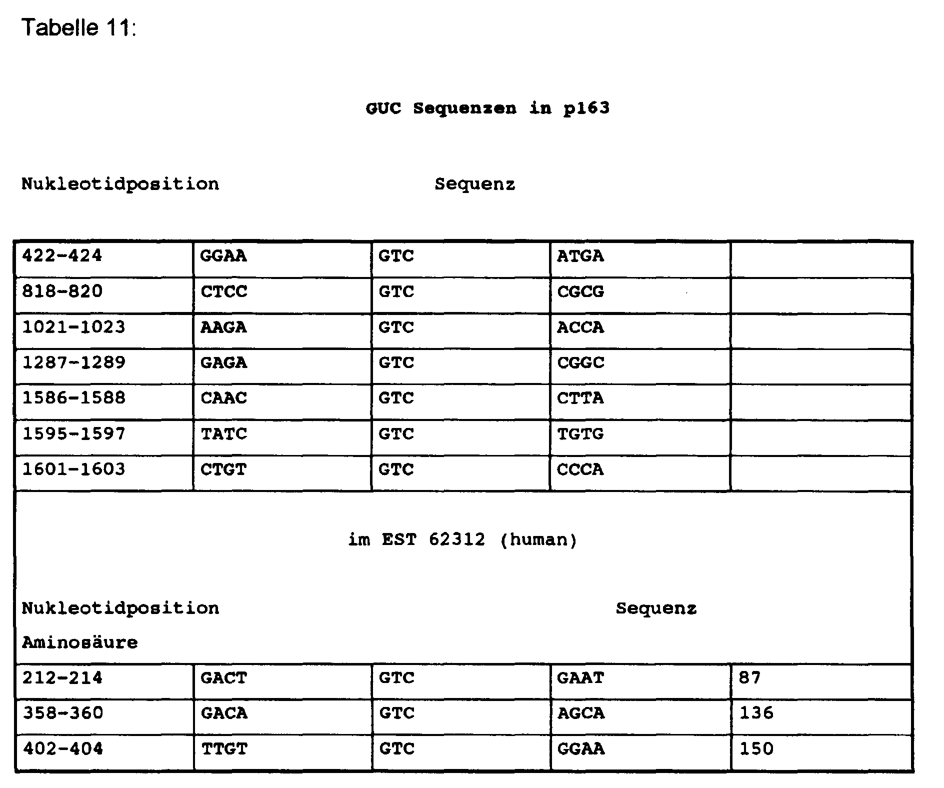

Die interagierende Domäne umfasst Aminosäuren 121-252 der Maussequenz, auf die sich die Numerierung bezieht. Dies entspricht den Nukleotiden 685-1078 der Maussequenz.

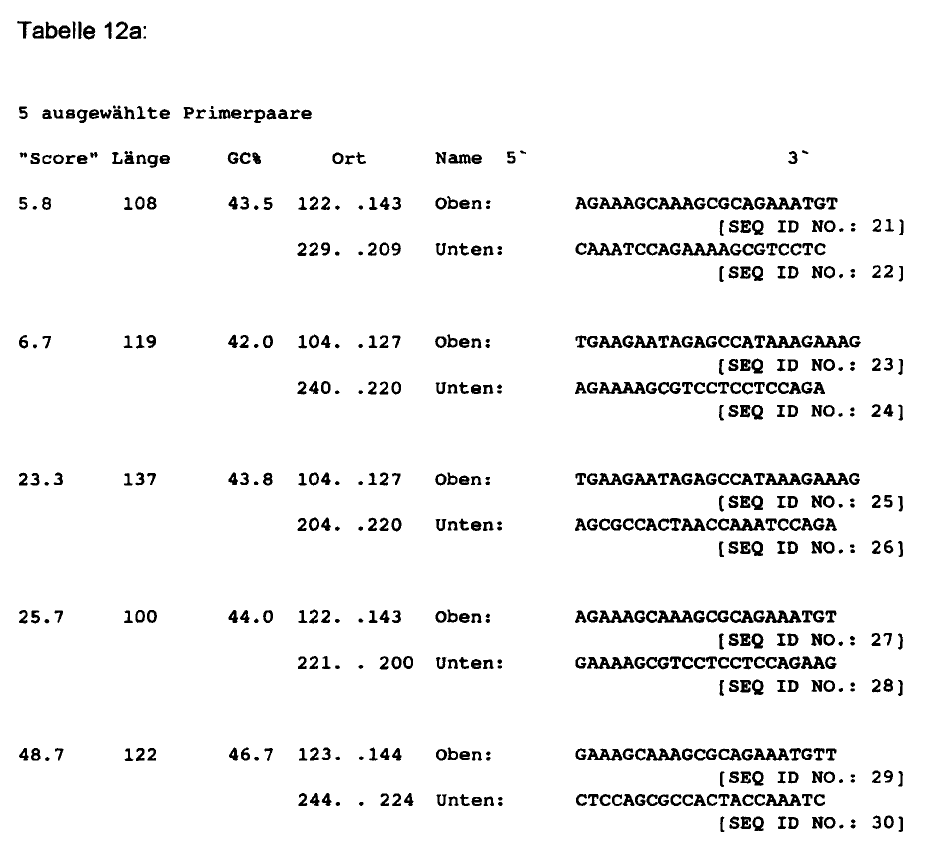

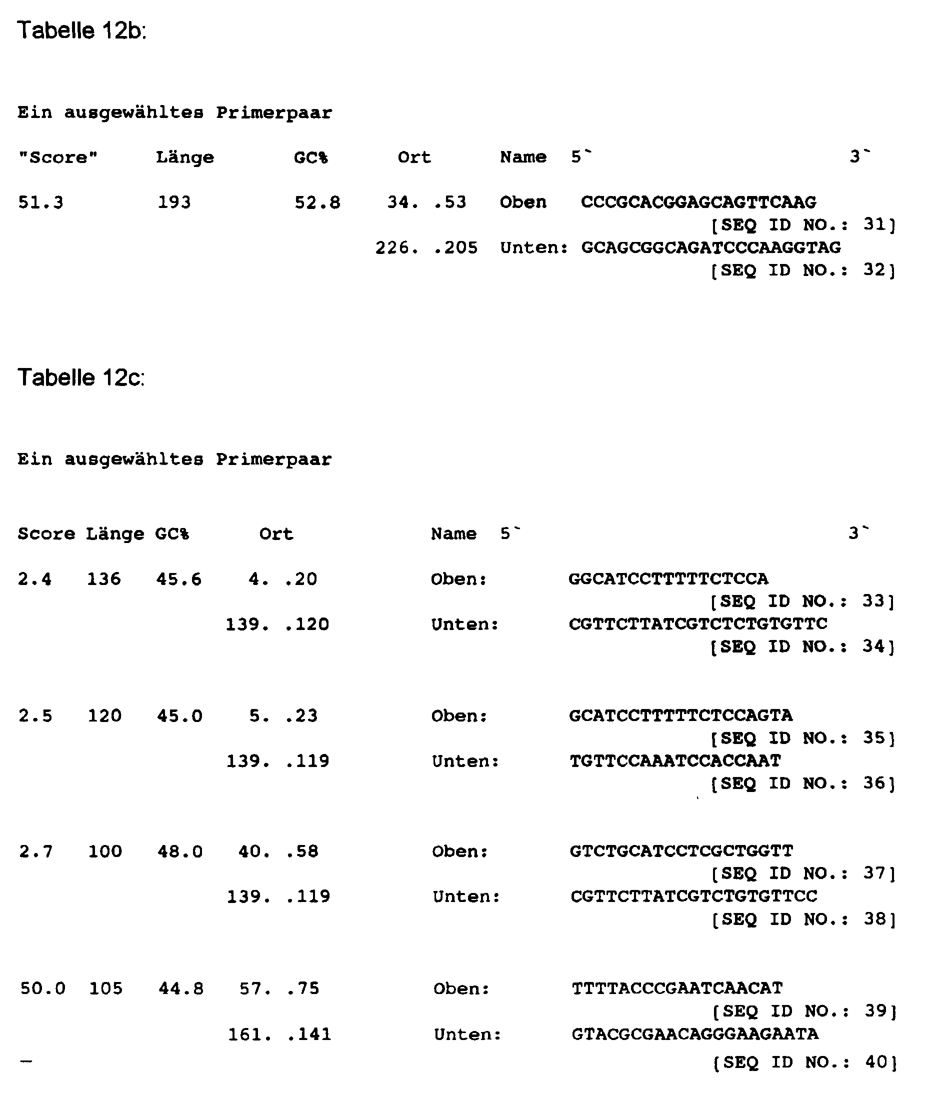

**Tabelle 13**

| Zusammenfassung der Wachstumsversuche | | | | |
|---|---|---|---|---|
| | Cyclin D1 | | Nup2 | |
| | nicht selektiv | selektiv | nicht selektiv | selektiv |
| wt p27 | +++ | +++ | +++ | +++ |
| Mut 106 | +++ | +++ | +++ | +++ |
| Mut 152 | +++ | + | +++ | + |
| Mut 294 | +++ | +++ | +++ | - |
| Mut 660 | +++ | +++ | +++ | - |
| Mut 687 | +++ | - | +++ | - |
| Mut 826 | +++ | +++ | +++ | +++ |
| Mut 850 | +++ | +++ | +++ | - |

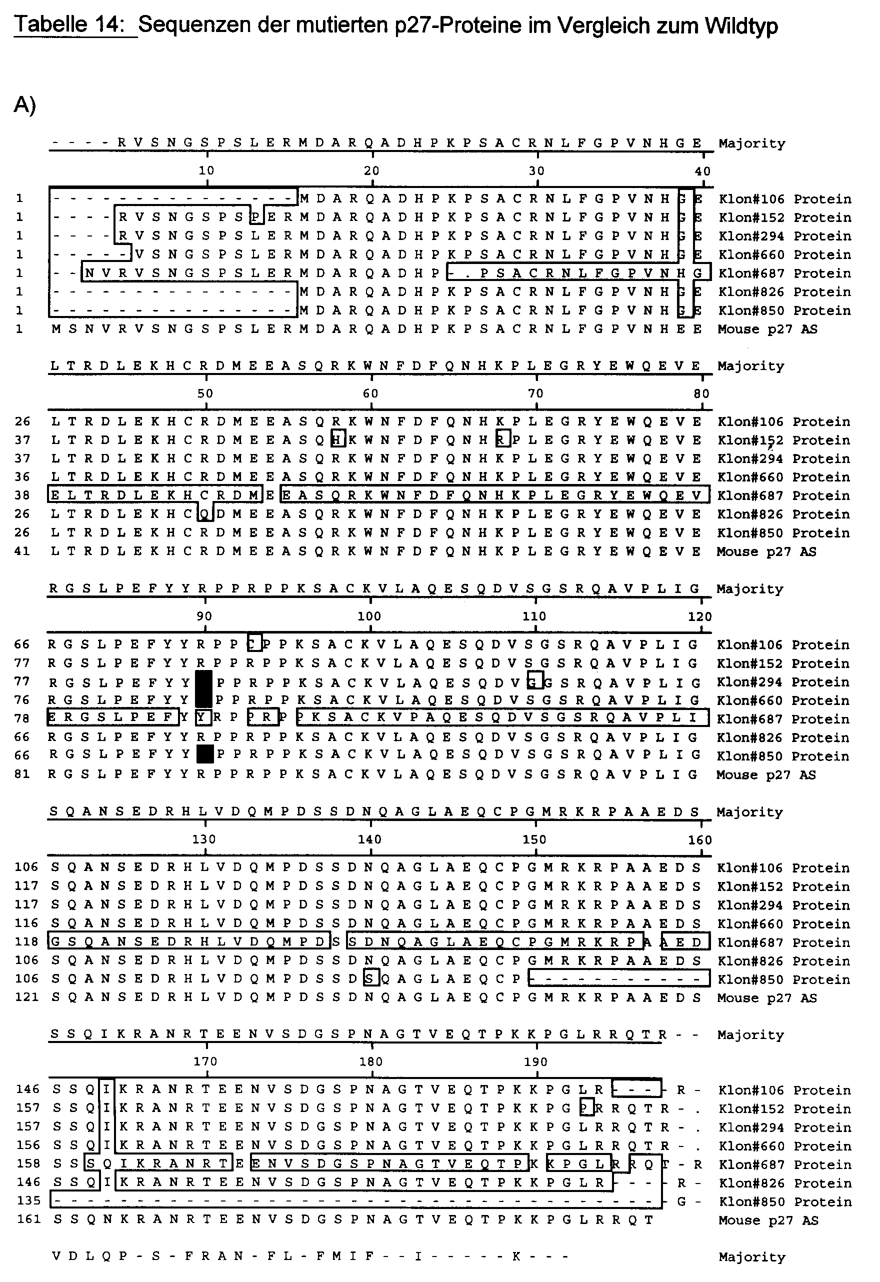

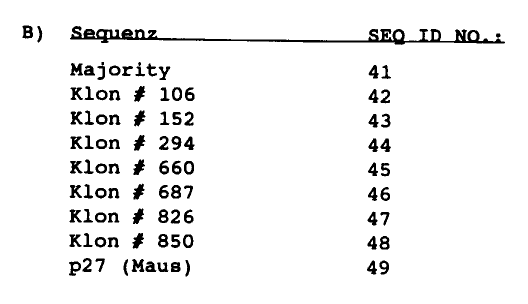

## Patentansprüche

1. Ein Protein, welches p27 inhibiert und hierdurch die durch das Protein p27 bewirkte Hemmung der Zellproliferation aufhebt.

2. Protein gemäß Anspruch 1, dadurch gekennzeichnet, daß es die Aminosäuresequenz von P163 gemäß Tabelle 6 [SEQ ID NO.: 16] oder Teile davon enthält.

3. Protein gemäß Anspruch 2, dadurch gekennzeichnet, daß die Teile der Aminosäuresequenz von p 163 ausgewählt werden aus der Gruppe enthaltend Peptide mit den Aminosäuresequenzen von Position 1 bis 24 [SEQ ID No.: 2], von Position 137 bis 196 [SEQ ID NO.: 4], von Position 215 bis 265 [SEQ ID NO.: 6], von Position 239 bis 272 [SEQ ID NO.: 8], von Position 399 bis 438 [SEQ ID NO.: 10] und von Position 307 bis 469 [SEQ ID NO.: 12] bezogen auf die Numerierung gemäß Tabelle 6.

4. Protein, welches durch Deletion funktioneller Domänen von dem Protein gemäß Anspruch 2 abgeleitet werden kann, dadurch gekennzeichnet, daß von der [SEQ ID NO. 16] der Tabelle 6 die p27-bindende Domäne oder die Ran-bindende Domäne deletiert ist.

5. Protein gemäß Anspruch 4, dadurch gekennzeichnet, daß nach Deletion der p27-bindenden Domäne ein Protein der Aminosäuresequenz gemäß Tabelle 8 [SEQ ID NO.: 18] und bei Deletion der Ran-bindenden Domäne ein Protein der Aminosäuresequenz gemäß Tabelle 9 [SEQ ID NO.: 19] vorliegt.

6. Protein, welches durch Deletion aller Aminosäuresequenzen außer dem Bereich der p27-bindenden Domäne von dem Protein gemäß Anspruch 2 abgeleitet werden kann.

7. Protein gemäß Anspruch 6, dadurch gekennzeichnet, daß es die Aminosäuresequenz gemäß Tabelle 10 [SEQ ID NO.: 20] enthält.

8. Protein gemäß einem oder mehreren der Ansprüche 1 bis 7, dadurch gekennzeichnet, daß es sich um das entsprechende homologe Protein oder die entsprechenden Teile davon, einer anderen Säugetierspezies oder des Menschen handelt.

9. Protein gemäß Anspruch 8, dadurch gekennzeichnet, daß es die Aminosäuresequenz des menschlichen Homologs des p163 enthält.

10. DNA, kodierend für ein Protein gemäß einem oder mehreren der Ansprüche 1 bis 9.

11. DNA gemäß Anspruch 10, dadurch gekennzeichnet, daß sie
(a) die Nukleotidsequenz gemäß Tabelle 3 [SEQ ID NO.: 1] oder Teile davon enthält,
(b) unter stringenten Bedingungen mit der unter (a) genannten Sequenz hybridisiert, oder
(c) gegenüber der unter (a) genannten Sequenz entsprechend der Degeneriertheit des genetischen Codes verändert ist, jedoch für die gleiche Aminosäuresequenz kodiert.

12. DNA gemäß Anspruch 11 zur Verwendung als PCR-Primer, dadurch gekennzeichnet, daß die Nukleotidsequenzen dieser PCR-Primer ausgewählt werden aus der Gruppe enthaltend die [SEQ ID NO: 21] bis [SEQ ID NO.: 30] der Tabelle 12a, die [SEQ ID NO.: 31] und [SEQ ID NO.: 32] der Tabelle 12b und die [SEQ ID NO.: 33] bis [SEQ ID NO.: 40] der Tabelle 12c.

13. Verwendung der DNA nach einem der Ansprüche 10 oder 11 zum Nachweis und/oder Quantifizierung der p163 mRNA in Zellen und/oder Geweben.

14. Verwendung gemäß Anspruch 13, dadurch gekennzeichnet, daß der Nachweis und/oder die Quantifizierung durch Northern Blotting erreicht wird.

15. Verwendung gemäß Anspruch 13, dadurch gekennzeichnet, daß der Nachweis und/oder die Quantifizierung durch PCR erreicht wird.

16. Verwendung gemäß Anspruch 15, dadurch gekennzeichnet, daß die PCR-Primer gemäß Anspruch 12 angewandt werden.

17. Verwendung gemäß Anspruch 13, dadurch gekennzeichnet, daß der Nachweis und/oder die Quantifizierung durch Fluoreszenz in situ Hybridisierung erreicht wird.

18. Verwendung des Proteins gemäß einem oder mehreren der Ansprüche 1 bis 9 zur Herstellung von Antikörpern, die an dieses Protein binden.

19. Antikörper und Spaltprodukte dieser Antikörper, welche an die Bindung für das p27-Protein des Proteins gemäß einem oder mehreren der Ansprüche 1 bis 9 binden.

20. Antikörper und Spaltprodukte dieser Antikörper, die an die Bindedomäne für das Ran-Protein des Proteins gemäß einem oder mehreren der Ansprüche 1 bis 9 binden.

21. Verwendung von Antikörpern gemäß der Ansprüche 19 oder 20 zum Nachweis des Proteins gemäß einem oder mehreren der Ansprüche 1 oder 2 in Zellen, Geweben oder Körperflüssigkeiten.

22. Antisense-Nukleinsäure, welche komplementär zu Teilbereichen der DNA gemäß einem der Ansprüche 10 oder 11, wobei diese Teilbereiche im Bereich der für die Aminosäuren 121 bis 467 kodierenden Nukleotide liegen.

23. Antisense-Nukleinsäure gemäß Anspruch 22, dadurch gekennzeichnet, daß die Antisense-Nukleinsäure ausgewählt wird aus der Gruppe enthaltend triple-DNA, synthetische Oligonukleotide, antisense RNA und Ribozyme.

24. Verwendung der Antisense-Nukleinsäure nach den Ansprüchen 22 oder 23 zur Hemmung der Proliferation einer Zelle dadurch charakterisiert, daß diese Zelle in vitro oder in vivo mit den Antisense-Nukleinsäure in Kontakt gebracht wird.

25. Nukleinsäurekonstrukt kodierend für Antisense-Nukleinsäure nach Anspruch 22 oder 23 zur Hemmung der Proliferation einer Zelle dadurch charakterisiert, daß eine DNA kodierend für die Antisense-Nukleinsäuresequenz mit mindestens einer Aktivierungssequenz verbunden wird und als nackte DNA, eingefügt in einen nichtviralen Vektor oder eingefügt in einen viralen Vektor, in die Zielzelle eingeführt wird.

26. Verwendung von Proteinen gemäß einem oder mehreren der Ansprüche 1 bis 9, dadurch gekennzeichnet, daß es zur Suche nach Inhibitoren der Wechselwirkung zwischen besagtem Protein und zellulären Bindungspartnern benutzt wird.

27. Verwendung gemäß Anspruch 26, dadurch gekennzeichnet, daß das "Two-Hybrid-System" verwendet wird.

28. Verwendung gemäß Anspruch 26, dadurch gekennzeichnet, daß ein Affinitätssystem benutzt wird, bei dem p163 oder die p27-bindende Domäne davon an eine feste Phase gebunden wird, diese feste Phase dann mit einer Prüfsubstanz inkubiert wird und schließlich mittels eines markierten Bindungspartners des Proteins gemäß einem oder mehreren der Ansprüche 1 bis 9 die Hemmung der Bindung des Bindungspartners mit dem genannten Protein ermittelt wird.

29. Verwendung gemäß Anspruch 28, dadurch gekennzeichnet, daß die Bindungspartner ausgewählt werden aus der Gruppe enthaltend p27 und Ran.

30. Verwendung eines Proteins gemäß einem oder mehreren der Ansprüche 3 bis 9 als Inhibitor der Funktion eines Proteins gemäß einem oder mehreren der Ansprüche 1, 2, 8 und 9.

31. Verwendung eines Antikörpers nach Anspruch 19 oder 20 als Inhibitor der Funktion eines Proteins gemäß einem oder mehreren der Ansprüche 1, 2, 8 und 9.

32. Nukleinsäurekonstrukt enthaltend eine DNA gemäß Anspruch 10 oder 11 operativ gekoppelt an eine Aktivierungssequenz, die die Expression eines Proteins gemäß einem oder mehreren der Ansprüche 1 bis 9 in einer Zelle ermöglicht.

33. Verwendung eines Nukleinsäurekonstruktes gemäß Anspruch 32 zur Stimulation der Zellproliferation.
